# FASCICULE DE BREVET EUROPEEN

(11) **EP 1 432 677 B1**
(45) Date de publication et mention de la délivrance du brevet: **13.02.2008**
(21) Numéro de dépôt: 02785516.2
(22) Date de dépôt: 02.10.2002
(51) Int. Cl.: C07C 275/14, A61P 31/00, A61P 35/00, A61K 31/17, C07C 275/24

(54) **OLIGOMERES D'UREES, LEUR PROCEDE DE PREPARATION ET COMPOSITIONS PHARMACEUTIQUES LES CONTENANT**
HARNSTOFFOLIGOMERE, DEREN ZUSAMMENSETZUNGEN UND VERFAHREN ZUR DEREN HERSTELLUNG
NOVEL UREA OLIGOMERS, THE PREPARATION METHOD THEREOF AND PHARMACEUTICAL COMPOSITIONS CONTAINING SAME

(30) Priorité: 02.10.2001 FR 0112659
(43) Date de publication de la demande: 30.06.2004
(73) Titulaire: CENTRE NATIONAL DE LA RECHERCHE SCIENTIFIQUE, 75794 Paris Cedex 16 (FR)
(72) Inventeur: GUICHARD, Gilles François Roger, 67202 Wolfisheim (FR); BRIAND, Jean-Paul, F-67000 Strasbourg (FR); SEMETEY, Vincent, F-15000 Aurillac (FR); NEUBERG, Patrick, L-9133 Schieren (LU)
(74) Mandataire: Grosset-Fournier, Chantal Catherine
(86) Numéro de dépôt international: PCT/FR2002/003355
(87) Numéro de publication internationale: WO 2003/029198

(56) Documents cités:
- WO-A-00/42009
- DD-A- 254 319
- TAMILARASU N ET AL: "Targeting RNA with peptidomimetic oligomers in human cells" BIOORGANIC & MEDICINAL CHEMISTRY LETTERS, OXFORD, GB, vol. 11, no. 4, 26 février 2001 (2001-02-26), pages 505-507, XP004230046 ISSN: 0960-894X

## Description

L'invention concerne de nouveaux oligomères d'urées, leurs procédés de préparation et les compositions pharmaceutiques les contenant.

La grande diversité fonctionnelle parmi les protéines est intimement liée à la structure tertiaire et/ou quaternaire extrêmement sophistiquée de ces dernières, mais elle repose sur un ensemble de briques structurales élémentaires extrêmement réduit, à savoir les hélices, les feuillets et les coudes, ces éléments structuraux étant eux-mêmes déterminés par la séquence en acides aminés, c'est-à-dire par la structure primaire de la protéine.

Récemment, les travaux de Seebach (Seebach, D. ; Matthews, J.L., Chem. Commun., 1997, 2015), de Gellman (Appela, D.H. et al., J. Am. Chem. Soc., 1997,118, 13071-13072) et d'Hannessian (Hanessian, S. et al., J. Am. Chem. Soc., 1998, 120, 8569-8570) ont révélé que des oligo-β ou γ-peptides (ω-peptides) de petite taille, composés exclusivement d'acides α-ou β-aminés étaient capables de former, en solution et en phase solide, des structures secondaires uniques, stables et prédictibles, de type hélice ou feuillet, voisines des structures protéiques. Dans la référence de Gellman (Gellman, S.H., Acc. Chem. Res., 1998, 31, 173-180), ces oligopeptides sont appelés des "foldamères".

La résistance de ces oligopeptides à la protéolyse en font de bons candidats prometteurs en chimie médicinale pour la découverte de nouvelles activités biologiques et pour interférer avec les processus de reconnaissance protéine-protéine.

A ce jour, il n'existe que très peu de données sur les propriétés structurales et biologiques d'oligomères énantiopurs ne renfermant pas de fonction amide dans leur squelette.

L'invention a pour but de fournir des molécules non peptidiques, comportant des chaînes latérales d'acides aminés, susceptibles d'adopter une structure hélicoïdale indépendante de la structure primaire desdites molécules, c'est-à-dire indépendante des chaînes latérales.

L'invention a également pour but de fournir des molécules susceptibles d'adopter une conformation hélicoïdale et capables de mimer les hélices naturelles actives.

L'invention a également pour but l'utilisation de ces nouveaux oligomères pour la préparation de médicaments dont les propriétés pharmacologiques sont notamment dues à l'interaction de ces oligomères avec les membranes cellulaires.

L'invention concerne l'utilisation des composés de formule générale :

X-(A)ₙ-Y, (I)

dans laquelle :
- n varie de 6 à 20,
- X représente un atome d'hydrogène, un groupe RₐCO, RₐOCO, RₐNHCO, RₐSO₂ ou RₐNHCS,
   Rₐ étant la fluorescéine ou un groupe alkyle (C1-C10), alkényle (C1-C10), alkynyle (C1-C10), aryle (C5-C12), aralkyle (C5-C14), hétéroaryle (C1-C5), lesdits groupes pouvant être non substitués ou substitués par 1 jusqu'à 6 substituants choisis parmi : un atome d'halogène, un groupe NO₂, OH, alkyle (C1-C4), NH₂, CN, trihalométhyle, acyloxy (C1-C4), dialkylamino (C1-C4), guanidino, un groupe SH, un groupe maléimide,
   sous réserve que X soit différent de H lorsque n est égal à 6,
- A représente indifféremment : un groupe 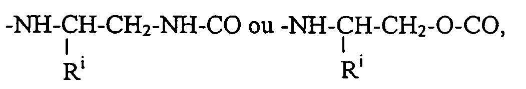
   * R' étant un atome d'hydrogène, une chaîne latérale d'acide aminé, un groupe alkyle (C1-C10), alkényle (C1-C10), alkynyle (C1-C10), aryle (C5-C12), aralkyle (C5-C14) ou hétéroaryle (C1-C10), lesdits groupes pouvant être non substitués ou substitués par 1 jusqu'à 6 substituants choisis parmi : un atome d'halogène, un groupe NO₂, OH, amidine, benzamidine, imidazole, alkoxy, alkyle (C1-C4), NH₂, CN, trihalométhyle, acyloxy (C1-C4), dialkylamino (C1-C4), guanidino,
   * i étant un nombre entier compris de 1 à n,
- Y est un groupe NR_{b}R_{c}, R_{b} et R_{c} ayant la signification donnée précédemment pour R',
pour la préparation de médicaments destinés au traitement de maladies bactériennes, fongiques ou cytotoxiques.

L'invention concerne également les composés de formule générale :

X-(A)ₙ-Y, (**I**)

dans laquelle :
- n varie de 6 à 20,
- X représente un atome d'hydrogène, un groupe RₐCO, RₐOCO, RₐNHCO, RₐSO₂ ou RₐNHCS,
   Rₐ étant la fluorescéine ou un groupe alkyle (C1-C10), alkényle (C1-C10), alkynyle (C1-C10), aryle (C5-C12), aralkyle (C5-C14), hétéroaryle (C1-C5), lesdits groupes pouvant être non substitués ou substitués par 1 jusqu'à 6 substituants choisis parmi : un atome d'halogène, un groupe NO₂, OH, alkyle (C1-C4), NH₂, CN, trihalométhyle, acyloxy (C1-C4), dialkylamino (C1-C4), guanidino, un groupe SH, un groupe maléimide,
   sous réserve que X soit différent de H lorsque n est égal à 6,
- A représente indifféremment :
   un groupe 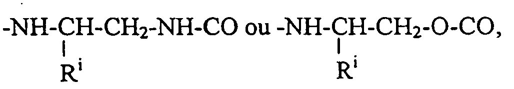
   * Rⁱ étant un atome d'hydrogène, une chaîne latérale d'acide aminé, un groupe alkyle (C1-C10), alkényle (C1-C10), alkynyle (C1-C10), aryle (C5-C12), aralkyle (C5-C14) ou hétéroaryle (C1-C10), lesdits groupes pouvant être non substitués ou substitués par 1 jusqu'à 6 substituants choisis parmi : un atome d'halogène, un groupe NO₂, OH, amidine, benzamidine, imidazole, alkoxy, alkyle (C1-C4), NH₂, CN, trihalométhyle, acyloxy (C1-C4), dialkylamino (C1-C4), guanidino,
   * i étant un nombre entier compris de 1 à n,
- Y est un groupe NR_{b}R_{c}, R_{b} et R_{c} ayant la signification donnée précédemment pour R',
sous réserve que les composés répondant aux formules suivantes soient exclus :

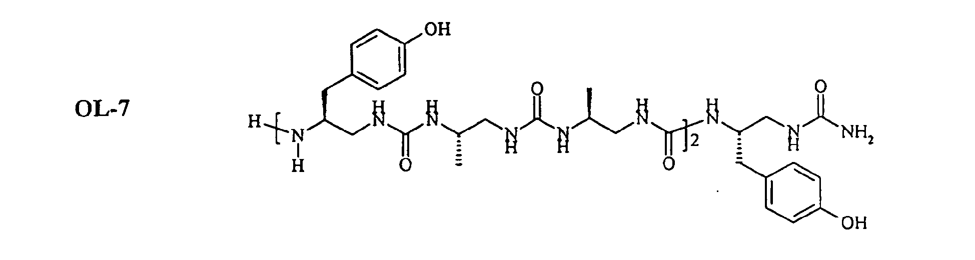

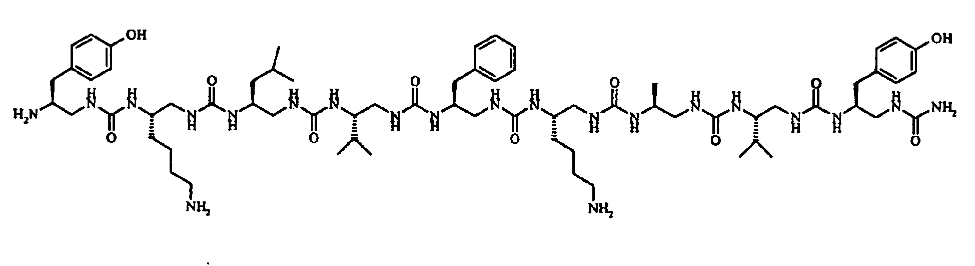

L'expression "A représente indifféremment un groupe 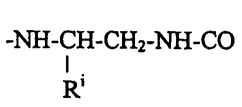 ou 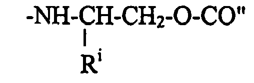 signifie que le groupe A, dans l'enchaînement (A)ₙ, est de manière aléatoire, l'un des groupes définis ci-dessus.

L'invention concerne les composés tels que définis ci-dessus, caractérisés en ce que n est égal à 6, 7, 8 ou 9, et répondant aux formules suivantes :

X-A³-A²-A³-A²-A²-A³-Y (13)

X-A²-A³-A²-A²-A³-A²-Y (14)

X-A³-A²-A³-A³-A²-A ³-A²-Y (15)

X-A³-A²-A³-A²-A²-A³-A²-Y (16)

X-A²-A³-A²-A²-A³-A²-A³-Y (17)

X-A²-A³-A²-A³-A³-A²-A³-Y (18)

X-A³-A²-A³-A²-A²-A³-A²-A³-Y (19)

X-A²-A³-A²-A³-A³-A²-A³-A²-Y (20)

X-A¹-A²-A³-A³-A¹-A²-A³-A¹-A³-Y (1)

X-A¹-A²-A³-A³-A¹-A²-A³-A³-A¹-Y (2)

X-A¹-A²-A³-A³-A¹-A³-A²-A¹-A³-Y (3)

X-A¹-A²-A³-A³-A²-A¹-A³-A¹-A³-Y (4)

X-A¹-A²-A³-A²-A¹-A³-A³-A¹-A³-Y (5)

X-A¹-A²-A³-A³-A²-A¹-A³-A²-A³-Y (6)

X-A¹-A²-A³-A¹-A²-A³-A¹-A²-A³-Y (7)

X-A²-A¹-A³-A²-A¹-A³-A²-A¹-A³-Y (8)

X-A¹-A²-A³-A³-A²-A³-A³-A²-A¹-Y (9)

X-A¹-A³-A²-A³-A²-A³-A²-A³-A¹-Y (10)

X-A¹-A²-A³-A²-A¹-A²-A³-A²-A¹-Y (11)

X-A³-A²-A³-A²-A²-A³-A²-A³-A³-Y (12)

dans lesquelles :
- X et Y sont tels que définis ci-dessus,
- A¹ correspond au groupe A tel que défini ci-dessus, dans lequel R' représente une chaîne latérale aromatique, telle qu'un groupe benzyle correspondant à la chaîne latérale de la phénylalanine, 4-hydroxybenzyle correspondant à la chaîne latérale de la tyrosine ou (3-indolyl)méthyl correspondant à la chaîne latérale du tryptophane,
- A² correspond au groupe A tel que défini ci-dessus, dans lequel R' représente une chaîne basique correspondant à la lysine (-(CH₂)₄-NH₂), l'arginine (-(CH₂)₃NH-C(=NH)NH₂) ou l'ornithine (-(CH₂)₃-NH₂), ou un groupe alkyle (C1-C10), alkényle (C1-C10), alkynyle (C1-C10), lesdits groupes pouvant être non substitués ou substitués par 1 jusqu'à 6 substituants choisis parmi : un groupe amidine, NH₂, guanidino,
- A³ correspond au groupe A tel que défini ci-dessus, dans lequel R' représente une chaîne latérale hydrophobe de type méthyle correspondant à la chaîne latérale de l'alanine, isopropyle correspondant à la chaîne latérale de la valine, isobutyle correspondant à la chaîne latérale de la leucine et sec-butyle correspondant à la chaîne latérale de l'isoleucine.

Des composés avantageux selon l'invention sont des composés tels que définis ci-dessus, répondant aux formules suivantes : 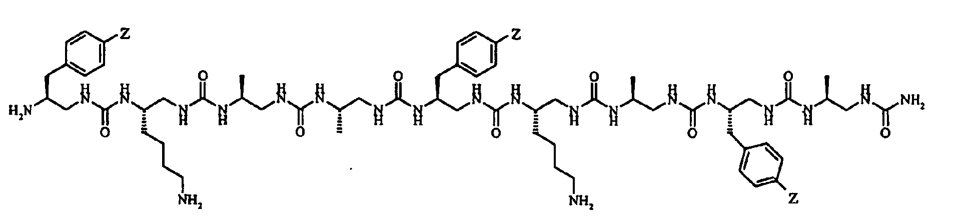 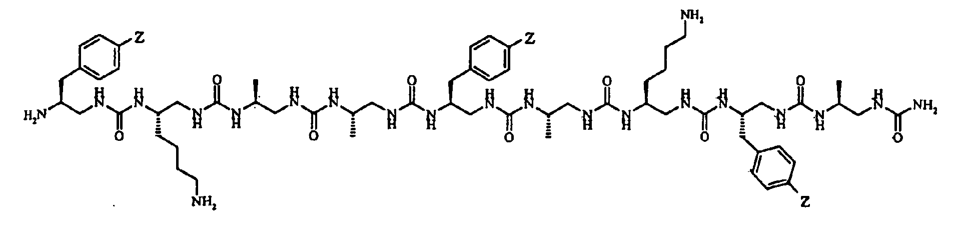 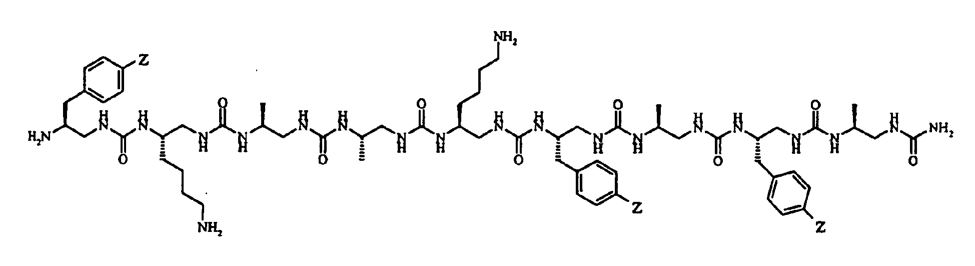 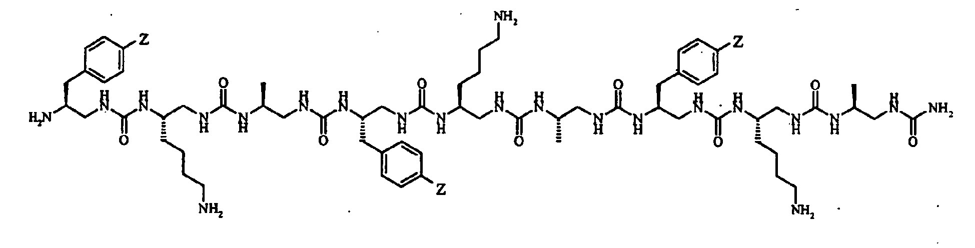 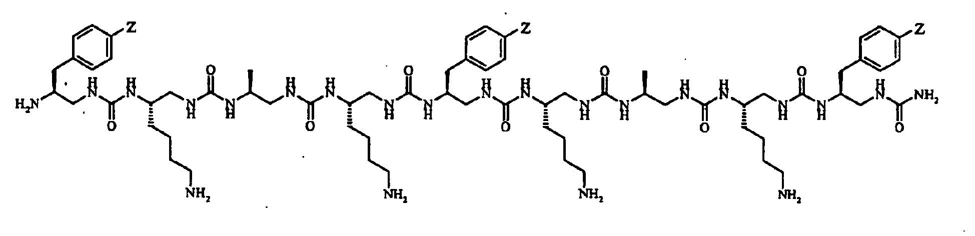 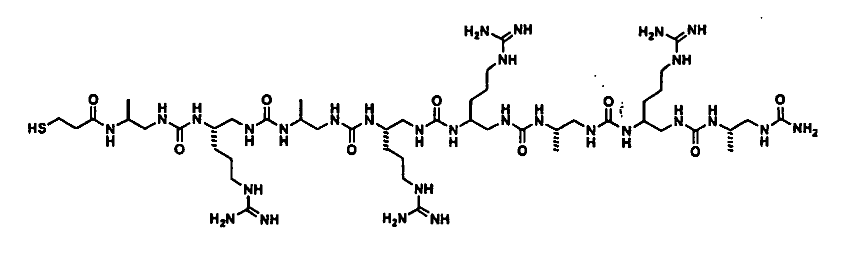 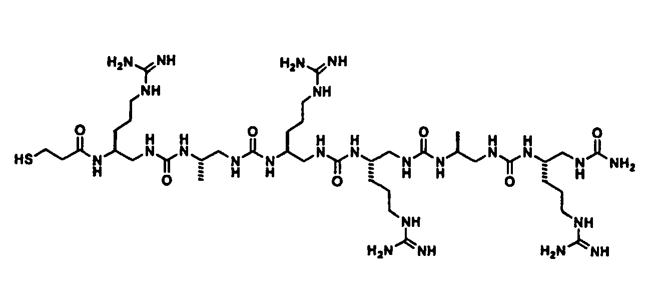 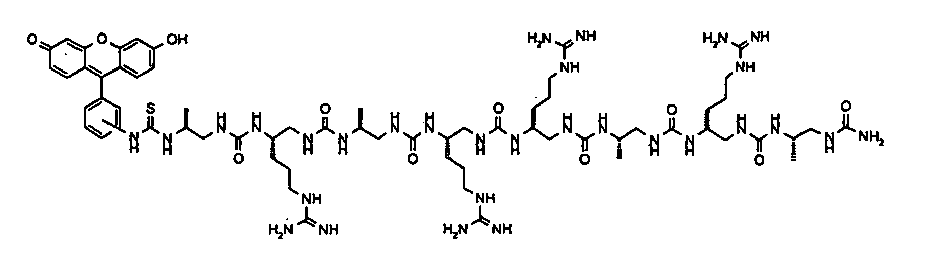 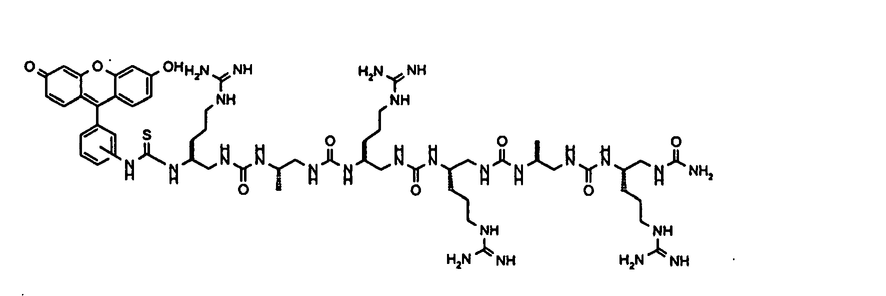 dans lesquelles Z représente soit un atome d'hydrogène soit un groupe OH.

L'invention concerne également des composés tels que définis ci-dessus, caractérisés en ce qu'au moins 10%, avantageusement de 10% à 50% des substituants R' sont des chaînes latérales d'acides aminés à caractère basique.

L'expression "chaînes latérales d'acides aminés à caractère basique" désigne des chaînes comportant au moins un ou plusieurs groupements ayant la possibilité d'accepter un proton, selon la définition de Bronsted. Par exemple, ces groupements peuvent être des amines (primaire, secondaire ou tertiaire), des amides, des amidines, des benzamidines, des guanidines, des imidazoles, des imidazolines ou des pyridines.

Des composés avantageux de l'invention sont des composés tels que définis ci-dessus, caractérisés en ce que les chaînes latérales d'acides aminés à caractère basique comportent des groupes amines primaire, secondaire ou tertiaire, des groupes imidazole, guanidine, amidine ou benzamidine.

Les molécules portant les chaînes latérales susmentionnées, à l'exclusion de toute chaîne chargée négativement de type aspartate ou glutamate, sont cationiques et sont donc attirées de manière électrostatique par les surfaces microbiennes chargées négativement. Dans le cas des bactéries Gram -, les molécules peuvent interagir initialement avec la paroi riche en lipopolysaccharide (LPS) de la membrane externe. Dans le cas des bactéries Gram +, la surface est chargée négativement à cause de la présence d'acides teichoïque et teichuronique et des groupements carboxyle des résidus d'acides aminés dans les peptidoglycanes. Une fois la membrane cytoplasmique atteinte, les molécules cationiques peuvent alors interagir avec les têtes chargées négativement des phospholipides de la paroi extérieure (Tossi et al., Biopolymers, 2000, **55**, 4-30).

L'invention concerne des composés possédant une structure en hélice et répondant à la formule générale suivante :

X-(A)ₙ-Y, (**I**)

dans laquelle :
- n varie de 6 à 20,
- X représente un atome d'hydrogène, un groupe RₐCO, RₐOCO, RₐNHCO, RₐSO₂ ou RₐNHCS,
   Rₐ étant un groupe alkyle (C1-C10), alkényle (C1-C10), alkynyle (C1-C10), aryle (C5-C12), aralkyle (C5-C14), hétéroaryle (C1-C5), lesdits groupes pouvant être non substitués ou substitués par 1 jusqu'à 6 substituants choisis parmi : un atome d'halogène, un groupe NO₂, OH, alkyle (C1-C4), NH₂, CN, trihalométhyle, acyloxy (C1-C4), dialkylamino (C1-C4), guanidino, un groupe SH, un groupe maléimide, la fluorescéine,
   sous réserve que X soit différent de H lorsque n est égal à 6,
- A représente indifféremment : un groupe 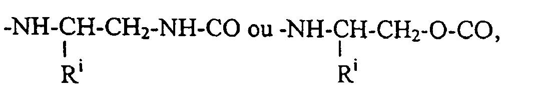
   * R' étant un atome d'hydrogène, une chaîne latérale d'acide aminé, un groupe alkyle (C1-C10), alkényle (C1-C10), alkynyle (C1-C10), aryle (C5-C12), aralkyle (C5-C14) ou hétéroaryle (C1-C10), lesdits groupes pouvant être non substitués ou substitués par 1 jusqu'à 6 substituants choisis parmi : un atome d'halogène, un groupe NO₂, OH, amidine, benzamidine, imidazole, alkoxy, alkyle (C1-C4), NH₂, CN, trihalométhyle, acyloxy (C1-C4), dialkylamino (C1-C4), guanidino,
   * i étant un nombre entier compris de 1 à n,
- Y est un groupe NR_{b}R_{c}, R_{b} et R_{c} ayant la signification donnée précédemment pour R',
   ladite hélice présentant les caractéristiques suivantes :

- elle est de pas régulier à droite, compris de 4,9 Å à 5,3 Å, et notamment égal à 5 Å,
- elle comprend de 2,4 à 2,6 résidus par tour et notamment 2,5 résidus par tour,
- son diamètre intérieur calculé à partir des centres des atomes est compris de 3,8 Å à 4,6 Å, et notamment égal à 4,2 Å,
- son diamètre intérieur calculé à partir de la surface de Van der Waals est compris de 1,4 Å à 1,8 Å, et notamment égal à 1,6 Å,

Par "résidu", on désigne le groupe A tel que défini ci-dessus.

L'expression "diamètre intérieur calculé à partir des centres des atomes" signifie que, dans une partie régulière de la structure, modélisée à partir des données obtenues par résonance magnétique nucléaire, on peut définir un diamètre entre deux atomes diamétralement opposés, en connaissant l'axe de l'hélice. La distance obtenue est alors mesurée à partir du centre des atomes.

L'expression "diamètre intérieur calculé à partir de la surface de Van der Waals" signifie que, dans une partie régulière de la structure, on peut définir, en connaissant l'axe de l'hélice, un diamètre entre deux atomes sélectionnés diamétralement opposés. La distance obtenue est alors la distance préalablement calculée diminuée des rayons de van der Waals des deux atomes sélectionnés. A titre d'exemple, on peut donner les rayons de van der Waals des atomes suivants : 1,2 Å pour l'atome d'hydrogène ; 1,5 Å pour l'atome d'oxygène ; 1,6 Å pour l'atome d'azote ; 1,7 Å pour l'atome de carbone et 1,8 Å pour l'atome de soufre.

On a constaté que la structure en hélice des composés de l'invention résulte en partie de l'existence de deux liaisons hydrogène entre l'atome d'oxygène du groupe CO du résidu i et les deux atomes d'hydrogène des groupes NH du résidu i+3 et N'H du résidu i+2 respectivement. Pour plus de clarté, l'atome d'azote situé entre le groupe CH₂ et le groupe CO est représenté par N'.

Ces liaisons hydrogène, entre, d'une part, l'atome d'oxygène du groupe CO du résidu i et l'atome d'hydrogène du groupe NH du résidu i+3, et, d'autre part, entre l'atome d'oxygène du groupe CO du résidu i et l'atome d'hydrogène du groupe N'H du résidu i+2, entraînent la formation de cycles à 12 ou 14 atomes. En effet, la liaison hydrogène entre le groupe CO du résidu i et l'atome d'hydrogène du groupe NH du résidu i+3 ferme un cycle à 14 atomes alors que la liaison hydrogène entre le groupe CO du résidu i et l'atome d'hydrogène du groupe N'H du résidu i+2 ferme un cycle à 12 atomes.

Il est possible de corréler la structure en hélice observée par RMN dans le méthanol et dans la pyridine avec un signal caractéristique en dichroïsme circulaire (DC).

Le dichroïsme circulaire représente une source d'information considérable sur la structure des macromolécules biologiques telles que les protéines ou l'ADN (G.D. Fasman, Circular Dichroism and the conformational analysis of biomolecules, Plenum press, NY, 1996). Le dichroïsme circulaire est la mesure en fonction de la longueur d'onde de la capacité d'une molécule optiquement active à absorber de manière différente les deux constituants (tournant à gauche (Eg) et tournant à droite (Ed)) d'un faisceau lumineux polarisé. L'activité optique est donc liée à l'anisotropie optique du milieu étudié. La combinaison de l'absorption différentielle (dichroïsme circulaire) et de la différence de vitesse de transmission de la lumière polarisée droite et gauche (activité optique) dans la région spectrale où se manifeste une bande d'absorption optiquement active est appelée effet Cotton (Pierre Crabbé, Applications de la dispersion rotatoire optique et du dichroïsme circulaire optique en chimie organique, Gauthier-Villars, Paris, 1968).

Dans le méthanol, on observe un effet *Cotton* intense qui correspond vraisemblablement à la forme hélicoïdale (maximum à 205 nm) pour l'heptamère et pour le nanomère dans le méthanol et une absence de signal pour des oligomères plus courts : un hexamère dont le résidu 1 n'est pas acylé (absence de groupe carbonyle pouvant être engagé dans une liaison hydrogène avec le résidu 3) ne donne quasiment pas de signal à cette longueur d'onde. Dans le cas de l'heptamère, l'étude du signal à 205 nm en fonction de la température indique que celui-ci est peu sensible à une augmentation de la température et suggère que la structure secondaire persiste jusqu'à 60°C et que le mécanisme de dépliement est non-coopératif (on obtient une droite de pente négative). En chauffant un composé, on déstabilise la structure petit à petit et vraisemblablement la dénaturation complète de la molécule passe par un grand nombre d'états intermédiaires partiellement repliés : on parle alors d'effet non-coopératif.

Dans le trifluoroéthanol, le signal caractéristique disparaît alors que dans l'eau une partie du signal à 205 nm subsiste, ce qui suggère que la structure en hélice est en partie présente dans l'eau.

On a également constaté qu'un minimum de six résidus était nécessaire afin d'obtenir un composé en hélice. Cependant, dans le cas de six résidus (n=6), il est avantageux voire nécessaire de protéger le groupe NH terminal par un groupe acyle afin de stabiliser la structure de l'hélice.

Le terme "composés possédant une structure en hélice" désigne à la fois des composés dont la structure hélicoïdale est totale et des composés dont la structure hélicoïdale est partielle.

Une structure hélicoïdale partielle est définie lorsque soit une partie seulement de la molécule adopte les caractéristiques structurales de l'hélice, soit lorsqu'il existe un équilibre dynamique entre la forme hélicoïdale et d'autres états conformationnels. Ceci peut s'observer par différentes techniques qui incluent la RMN en solution ou bien le dichroïsme circulaire. La stabilité de l'hélice peut également être mesurée par des études thermiques.

L'invention concerne également les composés tels que définis ci-dessus et répondant à l'une des formules suivantes :

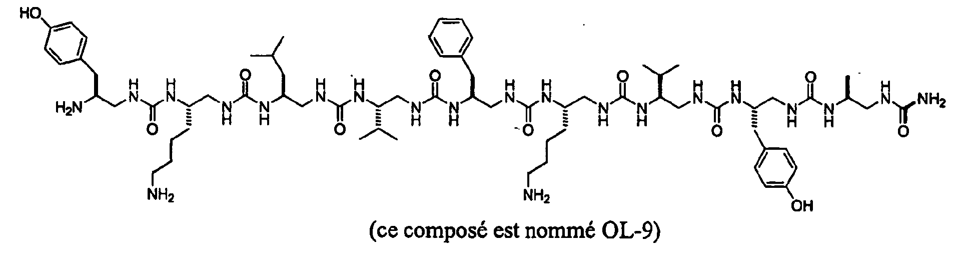

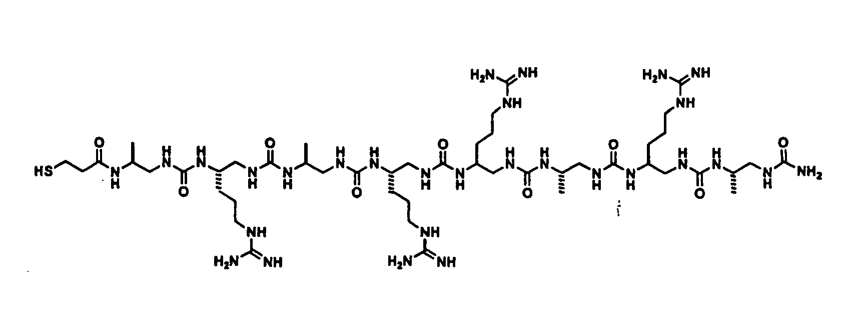

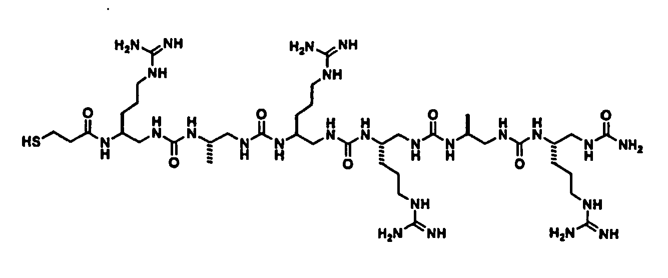

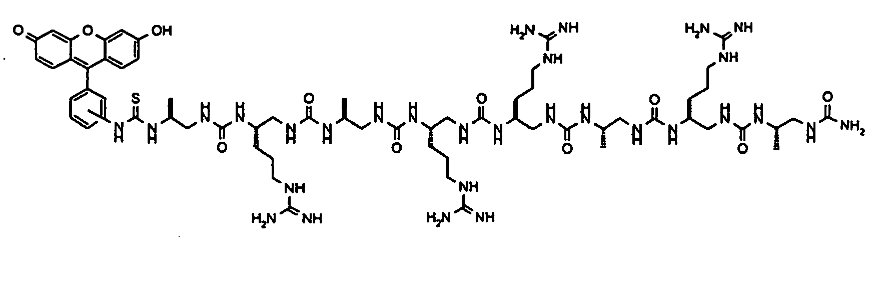

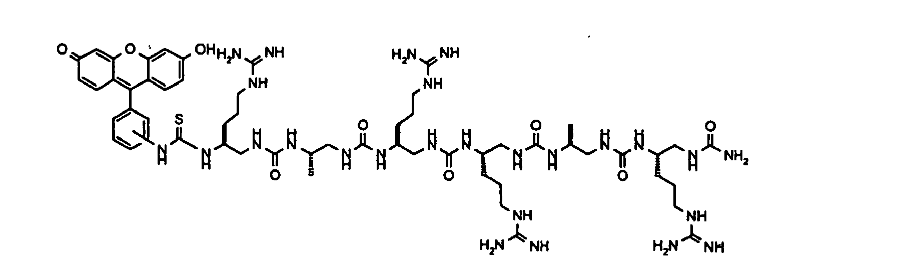

L'invention concerne également une composition contenant au moins un composé tel que défini ci-dessus et au moins un solvant, ledit solvant ayant la propriété de conférer aux molécules dudit composé une forme hélicoïdale, même partiellement, dans le cas d'un équilibre conformationnel entre les molécules dudit composé sous forme hélicoïdale et les molécules dudit composé sous forme non hélicoïdale, et étant notamment choisi parmi les alcools, la pyridine, l'acétonitrile, le diméthylsulfoxyde, l'eau en présence ou non de micelles ou de lipides, et étant avantageusement choisi parmi l'eau en présence ou non de micelles, le méthanol, l'éthanol, l'isopropanol, le trifluoroéthanol, l'hexafluoroisopropanol, le diméthylsulfoxyde ou tout mélange de ces solvants.

La structure est extrêmement stable et bien définie dans la pyridine ou bien dans le méthanol. Dans l'eau, les mesures effectuées par dichroïsme circulaire suggèrent que la structure hélicoïdale peut être partielle car le signal à 205 nm est moins intense que dans le méthanol. Cependant, la structure peut également être mesurée par des études thermiques.

L'invention concerne également un procédé de préparation en phase solide des composés de formule (I) tels que définis ci-dessus, à partir de monomères pré-activés de formule GP-A-W, dans laquelle :
- GP est un groupe protecteur choisi parmi Fmoc, Teoc, phtalimide, Alloc, BOC et Cbz,
- A est tel que défini ci-dessus,
- W est issu du groupe W-H choisi parmi : N-hydroxysuccinimide, phénol, pentafluorophénol, pentachlorophénol, p-nitrophénol, 2,4-dinitrophénol, 2,4,5-trichlorophénol, 2,4-dichloro-6-nitrophénol, hydroxy-1,2,3-benzotriazole, 1-oxo-2-hydroxydihydrobenzo-triazine (HODhbt), 7-aza-1-hydroxy-benzotriazole (HOAt), 4-aza-1-hydroxybenzo-triazole (4-HOAt), imidazole, tétrazole, et résine WANG, et est de préférence N-hydroxysuccinimide ou p-nitrophénol,
caractérisé en ce qu'il comprend les étapes suivantes :
a) une étape de couplage de GP-A-W tel que défini ci-dessus sur la fonction amine d'un support, ladite étape permettant le greffage du groupe -A-GP sur ladite fonction amine dudit support,
b) une étape de clivage du groupement GP dans des conditions appropriées,
c) la répétition séquentielle des étapes a) et b) jusqu'à ce que n groupes A aient été greffés sur ledit support,
d) une étape d'introduction des groupements RₐCO, RₐOCO, RₐNHCO ou RₐSO₂, Rₐ étant tel que défini ci-dessus,
e) une étape de clivage du composé de formule (I) du support par des moyens appropriés, qui permet le décrochage de la résine et la libération du groupe Y, tel que défini ci-dessus.

Par "monomère pré-activé", on désigne un dérivé d'acide carbamique ou d'acide carbonique capable de réagir avec des amines primaires ou secondaires, en présence ou non d'une base dans un solvant organique et généralement à température ambiante, pour donner respectivement une urée ou un carbamate.

Rappelons que le groupes protecteurs Fmoc, Teoc, Alloc, BOC et Cbz correspondent respectivement aux groupes fluorénylméthoxycarbonyle, triméthylsilyléthyloxycarbonyle, allyloxycarbonyle, tert-butyloxycarbonyle et benzyloxycarbonyle.

L'étape de couplage du procédé de préparation susmentionné en phase solide est effectuée en milieu organique (DMF, THF, CH₂Cl₂, NMP), par addition d'une solution du monomère préactivé (1 à 20 équivalents par rapport à la quantité d'amine libre sur la résine), en présence ou non d'un catalyseur, tel que l'hydroxybenzotriazole, à une suspension de résine dans le solvant. Cette étape peut être suivie ou non par l'adjonction d'une base organique (DIEA, NMM, pyridine, Et₃N, collidine, lutidine...).

L'étape de clivage est choisie en fonction du groupement protecteur GP. Dans le cas du groupement Fmoc, celui-ci peut être déprotégé par une solution à 20% de pipéridine dans le DMF. Dans le cas du groupement Teoc, celui-ci peut être déprotégé par une solution de fluorure de tétrabutylammonium dans du THF. Ces techniques sont classiques et sont largement décrites dans : "Protecting Groups" de P.J. Kocienski (Editions Thieme).

L'oligomère est ensuite allongé par répétition des étapes de couplage et de clivage, jusqu'au dernier résidu A de la séquence.

Après la déprotection du dernier groupement GP, les groupes RₐCO, RₐOCO, RₐNHCO ou RₐSO₂ peuvent être introduits, le cas échéant, par réaction de l'amine sur la résine avec par exemple un chlorure d'acyle (RₐCOCl), un anhydride mixte (RₐOCOORₐ), un isocyanate (RₐNCO) ou un chlorure de sulfanyle (RₐSO₂Cl) dans des conditions classiques.

L'étape de clivage peut être effectuée par traitement de la résine en milieu acide par des mélanges bien connus utilisés pour la synthèse peptidique, tel qu'un mélange TFA/H₂O/triisopropylsilane (9,5/2,5/2,5).

Un procédé de préparation avantageux selon l'invention est un procédé de préparation tel que défini ci-dessus, caractérisé en ce que l'étape a) peut être effectuée en présence ou non d'une base tertiaire telle que DIEA, collidine, NMM ou lutidine, et en présence ou non d'un catalyseur tel que HOBt.

Par définition, DIEA désigne la diisopropyléthylamine, NMM désigne la N-méthylmorpholine et HOBt le 1-hydroxybenzotriazole.

L'invention concerne également le procédé de préparation tel que défini ci-dessus, caractérisé en ce que l'étape a) peut être effectuée dans un solvant tel que le DMF, CH₂Cl₂, THF ou N-méthylpyrrolidone.

Un procédé de préparation avantageux selon l'invention est un procédé de préparation tel que défini ci-dessus, caractérisé en ce que le groupe GP est un groupe Fmoc.

L'invention concerne également un procédé de préparation en phase liquide des composés de formule (I) tels que définis ci-dessus, à partir de monomères pré-activés de formule GP-A-W, dans laquelle :
- GP est un groupe protecteur choisi parmi Fmoc, Alloc, BOC et Cbz,
- A est tel que défini ci-dessus,
- W est issu du groupe W-H choisi parmi : N-hydroxysuccinimide, phénol, pentafluorophénol, pentachlorophénol, p-nitrophénol, 2,4-dinitrophénol, 2,4,5-trichlorophénol, 2,4-dichloro-6-nitrophénol, hydroxy-1,2,3-benzotriazole, 1-oxo-2-hydroxydihydrobenzo-triazine (HODhbt), 7-aza-1-hydroxy-benzotriazole (HOAt), 4-aza-1-hydroxybenzo-triazole (4-HOAt), imidazole et tétrazole, et est de préférence N-hydroxysuccinimide ou p-nitrophénol,
caractérisé en ce qu'il comprend les étapes suivantes :
a) une étape de couplage de GP-A-W tel que défini ci-dessus sur la fonction amine du groupe A du dérivé H-A-Y,
b) une étape de clivage du groupement GP dans des conditions appropriées,
c) la répétition séquentielle des étapes a) et b) jusqu'à ce que n groupes A aient été greffés sur la fonction amine mentionnée ci-dessus,
d) une étape d'introduction des groupements RₐCO, RₐOCO, RₐNHCO ou RₐSO₂, Rₐ étant tel que défini ci-dessus,
e) une étape de clivage du composé de formule (I) par des moyens appropriés.

L'étape de couplage du procédé de préparation susmentionné en phase liquide est effectuée en milieu organique (DMF, THF, CH₂Cl₂, NMP), par addition d'une solution du monomère préactivé (1 à 1,5 équivalents par rapport à la quantité d'amine primaire ou secondaire du dérivé H-A-Y de départ). Cette étape peut être suivie ou non par l'adjonction d'une base organique (DIEA, NMM, pyridine, Et₃N, collidine, lutidine...). Cette étape est suivie par chromatographie sur couche mince. A la fin de la réaction, on ajoute une solution saturée d'hydrogénocarbonate de sodium, puis du dichlorométhane par exemple: La phase organique est lavée avec une solution saturée d'hydrogénocarbonate de sodium puis avec une solution de NaCl saturé. On peut éventuellement laver la phase organique en milieu acide (HCl 1N ou KHSO₄ 1N). La phase organique est récupérée, séchée sur MgSO₄ et le solvant est évaporé pour donner le composé attendu. Si le produit brut contient des impuretés, il peut être purifié par chromatographie sur silice avec un système de solvant approprié.

L'étape de clivage correspond à la déprotection du groupement protecteur GP. Dans le cas du groupement BOC, celui-ci peut être déprotégé par du TFA. Dans le cas du groupement Teoc, celui-ci peut être déprotégé par une solution de fluorure de tétrabutylammonium dans du THF. Ces techniques sont classiques et sont largement décrites dans "Protecting Groups" de P.J. Kocienski (Editions Thieme).

L'oligomère est ensuite allongé par répétition des étapes de couplage et de clivage, jusqu'au dernier résidu A de la séquence.

Après la déprotection du dernier groupement GP, les groupes RₐCO, RₐOCO, RₐNHCO ou RₐSO₂ peuvent être introduits, le cas échéant, par réaction de l'amine déprotégée avec par exemple un chlorure d'acyle (RₐCOCl), un anhydride mixte (RₐOCOORₐ), un isocyanate (RₐNCO) ou un chlorure de sulfanylc (RₐSO₂Cl) dans des conditions classiques. Le composé final peut être purifié par chromatographie sur silice (phase normale ou phase inverse).

L'invention concerne également un procédé de préparation des composés tels que définis ci-dessus, par mise en contact d'un des composés tels que définis ci-dessus ou d'un des composés de formule (VIIc) ou (VIId), avec un solvant tel que le méthanol, l'éthanol, le trifluoroéthanol, l'hexafluoroisopropanol, l'eau, ou avec des tampons biologiques, tels que des tampons phosphate salins ou non, ou avec un milieu micellaire ou lipidique.

Les composés de formule (VIIc) sont des composés nommés OL-7, et qui répondent à la formule suivante :

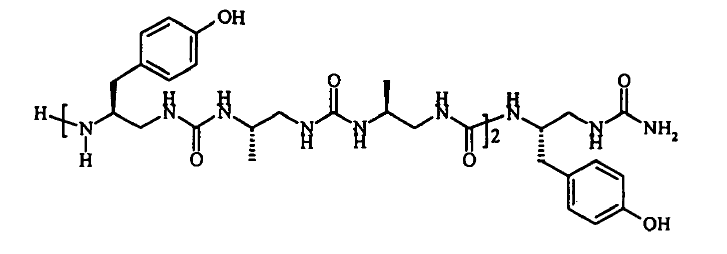

Les composés de formule (VIId) sont des composés répondant à la formule suivante :

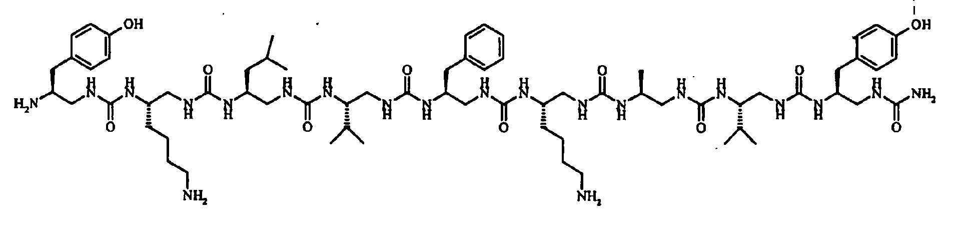

L'invention concerne l'utilisation des composés tels que définis ci-dessus pour la préparation de médicaments destinés au traitement de maladies bactériennes, fongiques ou cytotoxiques, et notamment d'infections fongiques telles que les aspergilloses et les candidoses, et d'infections bactériennes résistantes.

Par "maladies bactériennes", on désigne les infections émergentes associées aux bactéries suivantes :
- *Legionella,* bactérie associée à la légionellose,
- *Escherichia coli* O157:H7 : découverte en 1982, cette bactérie qui se transmet normalement par l'intermédiaire d'aliments contaminés, a été à l'origine de poussées de syndromes hémolytique et urémique,
- *Borrelia burgdorferi* : détectée aux Etats-Unis en 1982, elle a été identifiée comme étant la cause de la maladie de Lyme,
- *Vibrio cholerae* O139 : nouvelle souche associée à un choléra épidémique,
- *Helicobacter pyroli* : bactérie associée à un ulcère gastro-intestinal.

On désigne également les infections associées à des bactéries qui deviennent résistantes à une gamme de plus en plus étendue d'antibiotiques. Ainsi, dans de nombreuses régions, les antibiotiques de première intention, peu coûteux, ont perdu de leur efficacité contre les infections liées aux bactéries suivantes : *Escherichia coli, Streptococcus pneumoniae, Enterococcus faccalis, Mycobacterium tuberculosis, Pseudomonas aeruginosa, Neisseria gonorrhoca, Pneumococcus, Shigella, Staphylococcus aureus* (associé à un choc toxique staphylococcique) et *Moraxella catarrhalis*.

Par "maladies fongiques", on désigne les infections provoquées par les champignons pathogènes suivants : *Candida albicans*, associé à la candidose, *Aspergillus nidulans, Aspergillus parasiticus,* associés à l'aspergillose, et *Neurospora crassa*.

Les maladies fongiques comprennent les maladies causées par les champignons pathogènes, notamment ceux de la famille des fungi imperfecti, en particulier les moniliales ou encore ceux de la famille des hyprocréales ou de celle des sphaeriales.

Par "maladies cytotoxiques", on désigne en particulier les cancers, et notamment les tumeurs de l'appareil digestif (foie, intestin, oesophage, pancréas...), de l'appareil urogénital (utérus, prostate, rein, vessie...), des glandes endocrines, de l'oeil, de la peau, des seins, des os, du système nerveux, du thorax (poumon...).

L'invention concerne une composition pharmaceutique caractérisée en ce qu'elle comprend, à titre de substance active l'un au moins des composés tels que définis ci-dessus, en association avec un vecteur pharmaceutiquement acceptable.

Une composition pharmaceutique avantageuse selon l'invention est une composition pharmaceutique telle que définie ci-dessus, caractérisée en ce qu'elle contient par dose unitaire de 10 à 2000 mg de l'un des composés tels que définis ci-dessus.

Les compositions pharmaceutiques selon l'invention peuvent être administrées par voie orale, parentérale, rectale ou topique.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que l'amidon, la cellulose, le saccharose, le lactose ou la silice. Ces compositions peuvent également comprendre des substances autres, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylène glycol, des huiles végétales, en particulier l'huile d'olive, des esters organiques convenables. Ces compositions peuvent également contenir des adjuvants, en particulier des agents mouillants, isotonisants, émulsifiants, dispersants et stabilisants. La stérilisation peut se faire de différentes façons, par exemple par filtration aseptisante, en incorporant à la composition des agents stérilisants, par irradiation ou par chauffage. Elles peuvent être également préparées sous forme de compositions solides stériles, qui peuvent être dissoutes au moment de l'emploi dans un milieu stérile injectable.

Les compositions pour administration rectale sont les suppositoires ou les capsules rectales, qui contiennent, outre la substance active, des excipients tels que le beurre de cacao, des glycérides semi-synthétiques ou des polyéthylène glycols.

Les compositions stériles pour administration topique peuvent être par exemple des crèmes, pommades, lotions, collyres, collutoires, gouttes nasales ou aérosols.

Les modes d'administration préférés sont la voie parentérale et la voie orale.

Les doses pharmacologiques dépendent de l'effet recherché et de la durée du traitement; elles sont généralement comprises entre 0,001 mg/kg/jour et 100 mg/kg/jour, et de préférence entre 1 et 20 mg/kg/jour.

Ces quantités peuvent être administrées en une ou plusieurs doses.

Une composition pharmaceutique avantageuse selon l'invention est une composition pharmaceutique caractérisée en ce qu'elle comprend le composé tel que défini ci-dessus, nommé OL-9, en association avec un vecteur pharmaceutiquement acceptable.

Selon un autre mode de réalisation l'invention concerne également une composition pharmaceutique caractérisée en ce qu'elle comprend, à titre de vecteur pharmaceutique, l'un au moins des composés selon l'invention en association avec une substance pharmaceutiquement active.

L'invention concerne également l'utilisation d'un composé selon l'invention comme vecteur pharmaceutique.

Par « vecteur pharmaceutique » on désigne un composé selon l'invention favorisant le passage des substances avec lesquelles il se trouve en association au travers de membranes ou de téguments biologiques notamment la membrane cytoplasmique, l'enveloppe nucléaire ou la peau.

Certains oligomères de l'invention ont la capacité d'interagir avec les membranes et de pénétrer dans les cellules de mammifères *in vitro* et *in vivo.* Cette propriété peut en faire des composés intéressants pour faciliter la pénétration de molécules d'intérêt thérapeutique, comme les peptides ou les oligonucléotides, à travers des tissus notamment la peau, des membranes cellulaires ou encore à travers le noyau de ces cellules. Cette propriété est utile pour permettre d'augmenter la bio-disponibilité des peptides d'intérêt. Le peptide peut être associé à son transporteur de manière covalente ou être simplement mélangé avec celui-ci. Dans le cas d'une liaison covalente, un lien chimique potentiellement hydrolysable notamment une liaison ester, oxime ou amide ou un lien chimique pouvant être réduit notamment un pont disulfure sera préféré. La capacité du transporteur à assurer sa fonction est liée à la présence d'un certain nombre de résidus basiques, de préférence un groupement amine, guanidino ou benzamide. Un nombre avantageux de résidus assurant la fonction du transporteur est n = 4-10. Le nombre de résidus basiques sera avantageusement compris entre 40% et 100%.

En effet, il est connu de l'art antérieur que des peptides présentant un nombre minimal de résidus arginine consécutif (oligo-Arg) peuvent être internalisés dans les cellules et servir de transporteur à des molécules choisies, des peptides ou des principes actifs (P.A. Wender et al., Proc Natl Acad Sci USA 2000 Nov 21;97(24):13003-8). Il a été montré que 1a capacité de transport d'une molécule peut dépendre de la position relative des arginines dans la séquence peptidique (J. Rothbard et al. J Med Chem 2002 Aug 15;45(17):3612-8). Dans le cas d'une structure hélicoïdale, il a été montré que le positionnement des arginines sur une même face de l'hélice conduisant ainsi à une hélice amphipatique peut améliorer les propriétés de transport (A. Ho et al. Cancer Res 2001 Jan 15;61(2):474-7). La conception des composés avantageux de l'invention peut être rationalisée grâce à la connaissance de la structure hélicoïdale décrite dans l'invention et dont la projection dans un plan perpendiculaire a l'axe de l'hélice peut être utilisée pour visualiser le caractère amphipatique des composés. Un exemple de conception d'hélice amphipatique sur la base de la structure hélicoïdale 2.5 décrite dans l'invention est proposé ci-dessous. Les groupements A² et A³ sont définis comme précédemment, A² contenant préférentiellement un groupement guanidine, benzamidine ou amide et Pi indiquant la position du résidu dans la séquence :

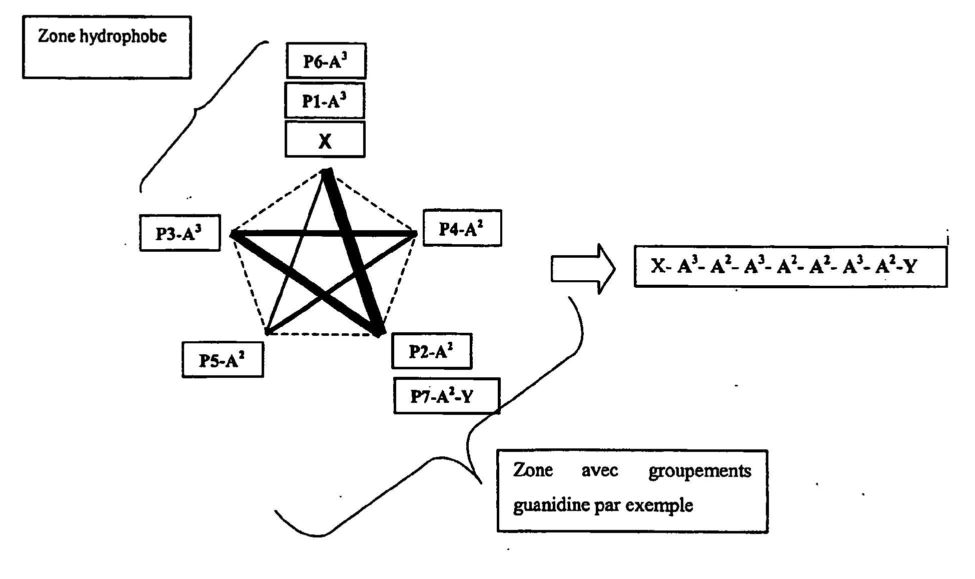

Les séquences suivantes définies précédemment peuvent être utilisées pour l'internalisation cellulaire :
- Héxamères

   X-A³-A²-A³-A²-A²-A³-Y (13)

   X- A²-A³-A²-A²-A³-A²-Y (14)
- Heptamères

   X- A³-A²-A³-A³-A²-A³-A²-Y (15)

   X-A³-A²-A³-A²-A²-A³-A²-Y (16)

   X- A²-A³-A²-A²-A³-A²-A³-Y (17)

   X- A²-A³-A²-A³-A³-A²-A³-Y (18)
- Octamère

   X- A³- A²- A³- A²- A²- A³- A²- A³- Y (19)

   X- A²- A³- A²- A³- A³- A²- A³- A²- Y (20)
- Nonamère

   X-A³-A²-A³-A²-A²-A³-A²-A³-A³-Y (12)

Le choix du groupement X est important et comporte avantageusement :
1) une fonctionalité permettant la ligation (liaison) ultérieure à la molécule (principe actif, peptide, oligonucléotide) qu'il s'agit de faire pénétrer dans les cellules.
   ou
2) le groupement X peut également être le produit de réaction du FITC (fluorescein isothiocyanate, produit commercial Ref. 46950 chez Fluka) sur l'amine du résidu suivant. Ce dérivé est utile pour prouver que l'oligomère étudié possède bien des propriété d'internalisation cellulaire.

### 4 exemples sont représentés ci-dessous :

Choix 1) groupement thiol permettant l'ancrage à une autre molécule comportant elle-même un groupement thiol ou nucléimide :

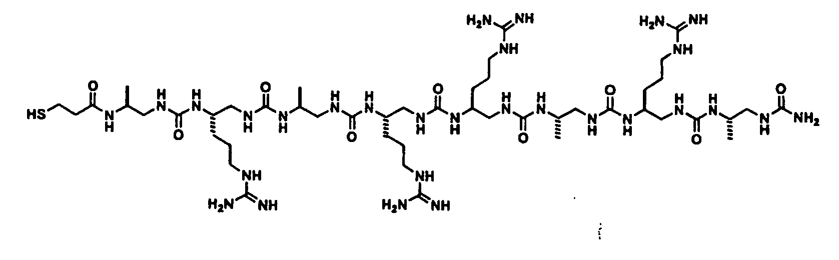

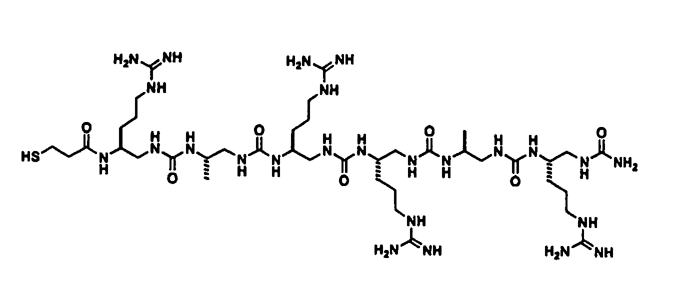

Choix 2) fonction fluorescéine pour un test d'internalisation cellulaire :

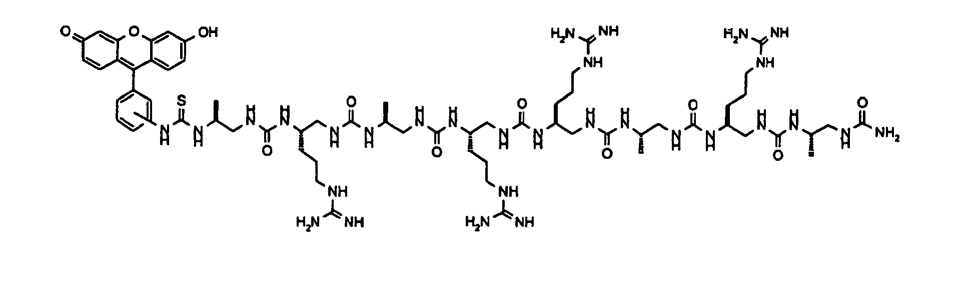

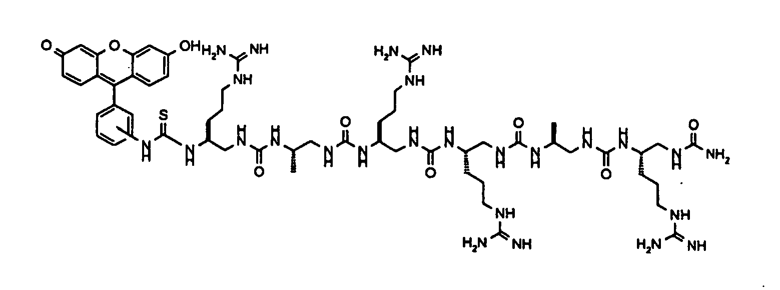

### DESCRIPTION DES FIGURES

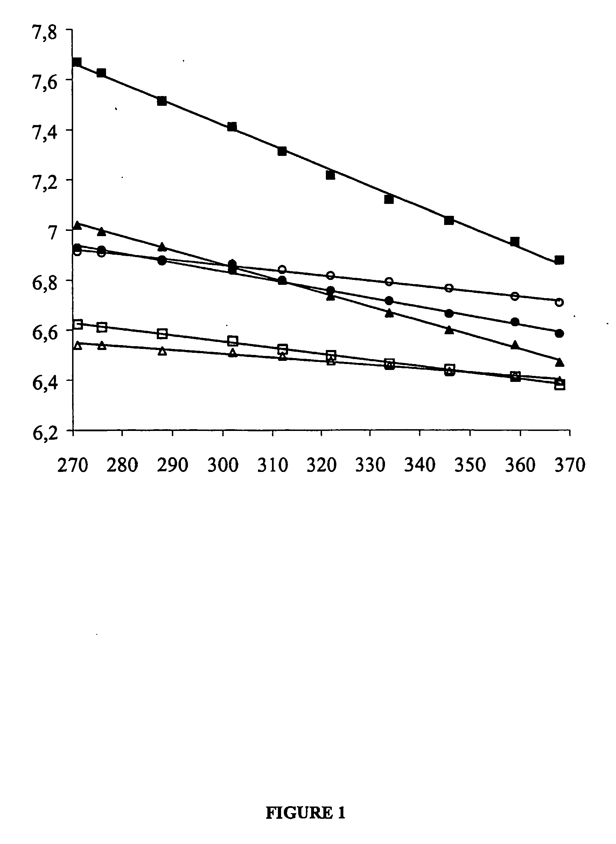

La **Figure 1** représente la variation des déplacements chimiques des protons NH de l'oligomère OL-7 en fonction de la température (en K).

Rappelons la formule d'un résidu pour les oligomères selon l'invention :

-NH-CHRⁱ-CH₂-N'H-CO-

La courbe avec les carrés noirs correspond au proton du résidu 2 (Ala) ; la courbe avec les triangles noirs correspond au proton du résidu 3 (Val) ; la courbe avec les ronds noirs correspond au proton du résidu 4 (Tyr) ; la courbe avec les carrés blancs correspond au proton du résidu 5 (Ala) ; la courbe avec les triangles blancs correspond au proton du résidu 6 (Val) ; la courbe avec les ronds blancs correspond au proton du résidu 7 (Tyr).

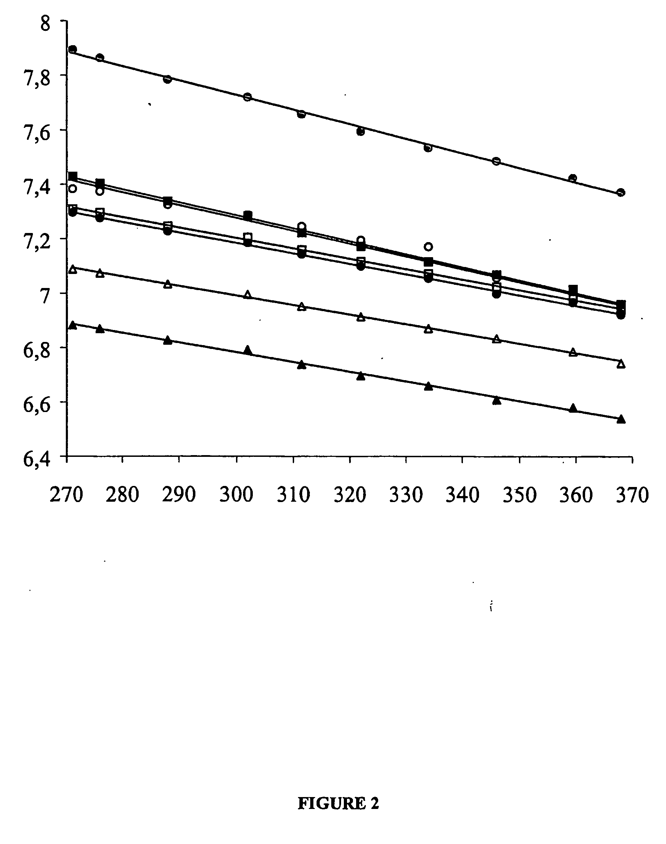

La **Figure 2** représente la variation des déplacements chimiques des protons N'H de l'oligomère OL-7 en fonction de la température (en K). La courbe avec les ronds gris correspond au proton du résidu 1 (Tyr) ; la courbe avec les carrés noirs correspond au proton du résidu 2 (Ala) ; la courbe avec les triangles noirs correspond au proton du résidu 3 (Val) ; la courbe avec les ronds noirs correspond au proton du résidu 4 (Tyr) ; la courbe avec les carrés blancs correspond au proton du résidu 5 (Ala); la courbe avec les triangles blancs correspond au proton du résidu 6 (Val) ; la courbe avec les ronds blancs correspond au proton du résidu 7 (Tyr).

Pour chacune des courbes des Figures 1 et 2, une régression linéaire a été effectuée. Ces régressions linéaires permettent d'obtenir la pente des droites et correspondent aux coefficients de température (voir tableaux 1 et 2). Ces valeurs renseignent sur l'accessibilité du proton vis-à-vis du solvant et de son engagement dans des liaisons hydrogène. Plus la valeur absolue de ce chiffre est faible (en particulier inférieure à 4), plus le proton est accessible au solvant et plus il est susceptible d'être engagé dans une liaison hydrogène. Ces mesures permettent donc de vérifier que les protons centraux de l'oligomère sont plus engagés dans une liaison hydrogène que les protons terminaux.

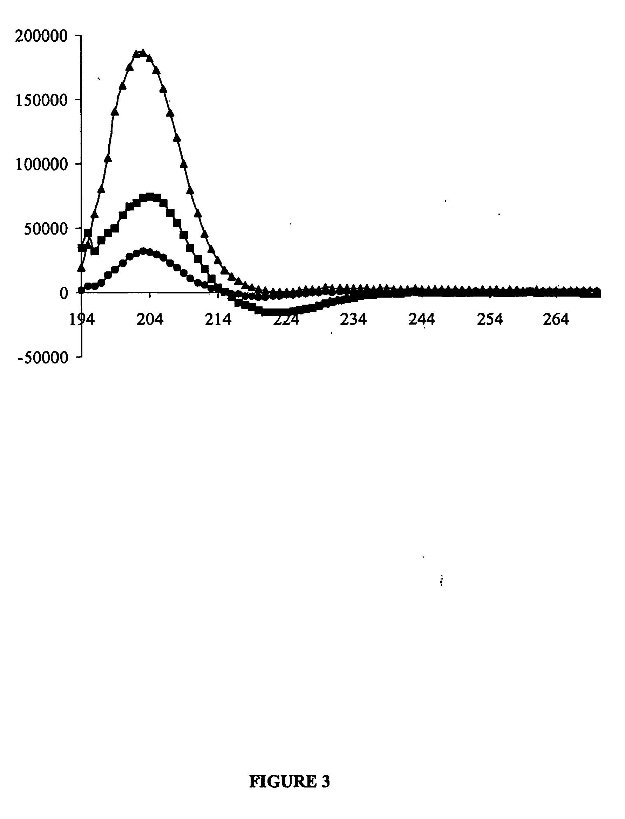

La **Figure 3** représente l'étude par dichroïsme circulaire du composé OL-7 dans différents solvants, à savoir dans le méthanol, dans l'eau et dans le trifluoroéthanol. Les courbes de cette figure représentent θ en fonction de la longueur d'onde en nm.

θ est l'ellipticité molaire et est exprimé en °.cm².dmol⁻¹.

θ peut être positif ou négatif et sa valeur est reliée à la structure secondaire des oligomères. Ce phénomène (effet Cotton) est extrêmement bien connu pour les peptides et pour d'autres types d'oligomères non-naturels.

La courbe avec les triangles noirs correspond au composé OL-7 étudié dans le méthanol ; la courbe avec les ronds noirs correspond au composé OL-7 étudié dans le trifluoroéthanol et la courbe avec les carrés noirs correspond au composé OL-7 étudié dans l'eau.

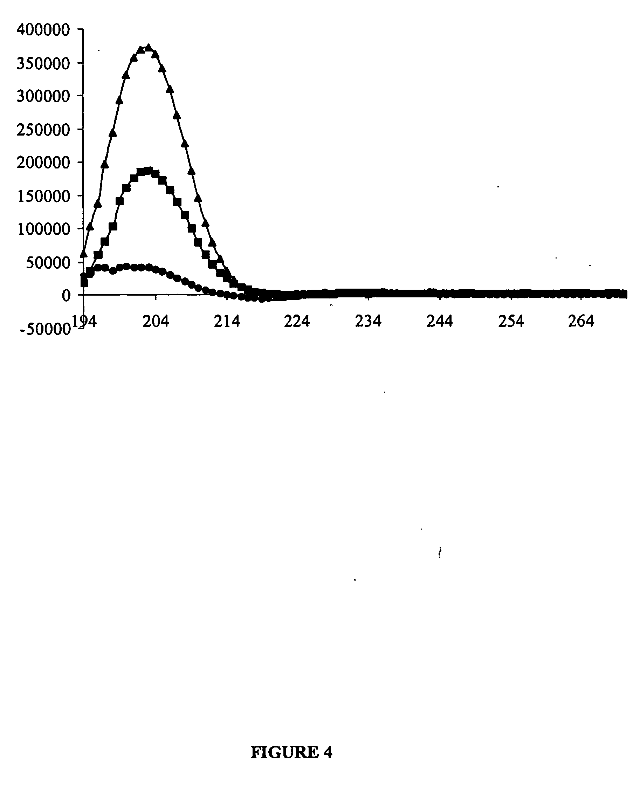

La **Figure 4** représente l'étude par dichroïsme circulaire des composés OL-6 (hexamère), OL-7 et OL-9 dans le méthanol.

Le composé OL-6 répond à la formule suivante :

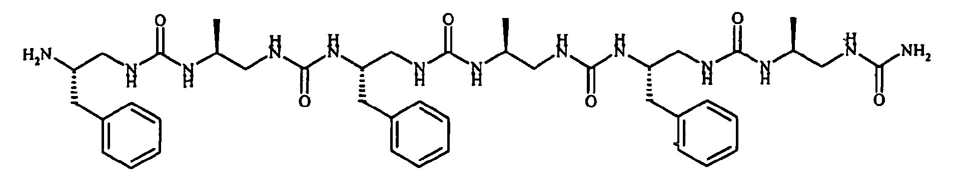

Les courbes de cette figure représentent θ (ellipticité molaire en °.cm².dmol⁻¹) en fonction de la longueur d'onde en nm.

La courbe avec les ronds noirs correspond au composé OL-6 ; la courbe avec les carrés noirs correspond au composé OL-7 et la courbe avec les triangles noirs correspond au composé OL-9.

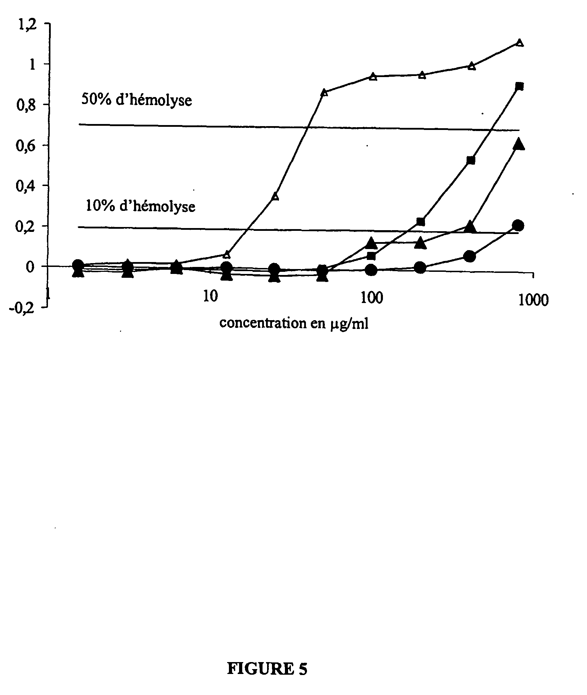

La **Figure 5** représente les résultats des tests d'hémolyse effectués sur le composé OL-9 selon l'invention.

Ce graphique représente la densité optique en fonction de la concentration en µg/ml du composé testé.

La courbe avec les triangles blancs correspond au chlorure de cétylpyridinium (ce composé sert de contrôle interne) ; la courbe avec les carrés noirs correspond au peptide de contrôle D ; la courbe avec les triangles noirs correspond au peptide de contrôle L et la courbe avec les ronds noirs au composé OL-9.

Le peptide de contrôle D est représenté par la séquence suivante :
H-DTyr-DLys-DLeu-DVal-DPhe-DLys-DAla-DVal-DTyr-NH₂
Le peptide de contrôle L est représenté par la séquence suivante :
H-Tyr-Leu-Val-Phe-Lys-Ala-Val-Tyr-NH₂

### EXEMPLES

### PREPARATION DES COMPOSES DE L'INVENTION NOTAMMENT OL-9 ET OL-7

Les oligomères de l'invention et notamment OL-9 et OL-7 ont été synthétisés en phase solide à partir d'une résine Rink amide (4-(2', 4'-Diméthoxyphényl-Fmoc-aminométhyl)phénoxyacétamido-4-méthylbenzhydrylamine résine) commerciale sur une échelle de 50µmol à partir des carbamates de succinimidyle 1 portant les chaînes latérales de Ala, Val, Leu, Phe, Tyr(tBu), Lys(Boc), Arg(PMC) ou Arg(Pbf) ; (préparés selon Guichard et. al., Tetrahedron Lett., 2000, 41, 1553-1557).

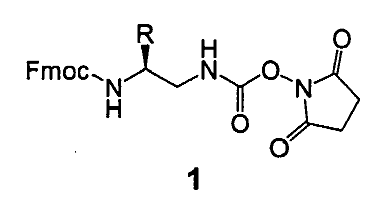

| **1** | R |
|---|---|
| **a** | Me |
| **b** | *i*Pr |
| **c** | iBu |
| **d** | Bn |
| **e** | Bn(OtBu) |
| **f** | (CH₂)₄NHBoc |

Le carbamate 1 désiré (4 équivalents) dans 2ml de DMF est ajouté à une suspension de la résine susmentionnée dans du DMF (2 ml) suivi par de la N-méthylmorpholine (1 équivalent). On laisse la réaction s'effectuer pendant 90 minutes et on la recommence, après filtration de la résine. Le groupement Fmoc est ensuite clivé par traitement avec 20% de pipéridine dans du DMF. Les techniques de lavage et de filtration de la résine ainsi que de déprotection du groupement Fmoc sont celles couramment utilisées en synthèse peptidique en phase solide. L'ensemble de l'opération (couplage et déprotection du Fmoc) est recommencée plusieurs fois avec les carbamates 1 possédant les chaînes latérales des résidus suivants dans la séquence pour donner après clivage de la résine (TFA, eau/triisopropylsilane : clivage standard utilisé en synthèse peptidique en phase solide en stratégie Fmoc) les produits OL-7 et OL-9 bruts, avec respectivement une pureté de 65% et 32% (déterminées par HPLC). Le produit pur est caractérisé par spectrométrie de masse (MALDI-MS) et par HPLC.

### RESULATS

### OL-7:

- Temps de rétention HPLC : 14,58 min (A : 0,1% TFA dans H₂O ; B : 0,08% TFA dans CH₃CN, 5-65% B en 20 minutes) (TFA= acide trifluoroacétique).
- MS (MALDI-TOF) : 1051,5 [M+H]⁺

### OL-9 :

- Temps de rétention HPLC : 15,2 min (A : 0,1% TFA dans H₂O ; B : 0,08% TFA dans CH₃CN, 5-65% B en 20 minutes) (TFA= acide trifluoroacétique).
- MS (MALDI-TOF) : 1393,1 [M+H]⁺

L'étude conformationnelle des deux oligomères OL-7 et OL-9, synthétisés en phase solide, a été effectuée par Résonance Magnétique Nucléaire (RMN) mono et bidimensionnelle initialement dans la pyridine-*d*₅ (dans la pyridine, les signaux des protons NH sont mieux dispersés que dans le chloroforme ou le méthanol, et l'attribution des signaux en est facilitée) puis dans le méthanol deutéré CD₃OH.

L'attribution des résonances ainsi que la détermination de la séquence a été effectuée par des expériences DQF-COSY, TOCSY et ROESY.

On rappelle par définition que DQF-COSY (Double Quantum filtered correlated spectroscopy), TOCSY (Total Correlated Spectroscopy) et ROESY (Rotating frame overhauser effect spectroscopy) sont des techniques classiques de spectroscopie.

Un certain nombre de données importantes pour la détermination de la structure secondaire ont été extraites des spectres monodimensionnels et des expériences COSY.

Les tableaux 1 et 2 ci-après indiquent les coefficients de température (en ppb.K⁻¹) calculés pour les protons des groupes NH et N'H des résidus 1 à 7 du composé OL-7 (voir Figures 1 et 2), et des résidus 1 à 9 du composé OL-9.

**Tableau 1**

| **résidu** | **N'H** | **NH** |
|---|---|---|
| 1 | - 5,3 | |
| 2 | - 4,8 | - 8,2 |
| 3 | - 3,6 | - 5,6 |
| 4 | - 3,8 | - 3,6 |
| 5 | - 3,8 | - 2,5 |
| 6 | - 3,5 | - 1,5 |
| 7 | - 4,7 | - 2,1 |

**Tableau 2**

| **résidu** | **N'H** | **NH** |
|---|---|---|
| 1 | - 7,8 | |
| 2 | - 6,7 | - 8,7 |
| 3 | - 3,25 | - 5,2 |
| 4 | - 5 | - 2,4 |
| 5 | - 5 | -0,8 |
| 6 | - 4,2 | - 1,2 |
| 7 | - 4,75 | - 1,88 |
| 8 | -3,75 | - 1,1 |
| 9 | - 5 | - 2,5 |

Ces chiffres sont établis à partir des courbes de déplacements chimiques en fonction de la température en calculant les pentes des droites obtenues par régression linéaire.

Ces coefficients de température s'expriment en ppb.K⁻¹ et sont notés δΔ/δT.

La mesure des coefficients de température (chauffage jusqu'à environ 100°C pour OL-7) sur ces deux molécules suggère que les protons NH des résidus 4-7 pour OL-7 et 4-9 pour OL-9 sont peu accessibles au solvant et sont potentiellement liés par liaison hydrogène (-Δδ/ΔT < 2,5 pour le nonamère) (à l'exception en général des deux résidus N-terminaux pour lesquels les coefficients de température sont supérieurs à 5). Les protons N'H des résidus centraux ont des coefficients de températures plus négatifs (3,5 <-Δδ/ΔT < 3,8 pour les résidus 3-7 dans le cas de l'oligomère OL-7) ; ils sont donc vraisemblablement plus accessibles au solvant et donc moins engagés dans des liaisons hydrogène que chacun des NH correspondants au sein de chaque fonction urée.

De plus les constantes de couplage *J*(NH, ^{β}CH) sont élevées (à l'exception des deux résidus N-terminaux, les constantes sont de l'ordre de 9-10 Hz) et peu sensibles aux changements de température (voir tableaux 3 et 4 ci-après).

**Tableau 3.** Etude de la variation des constantes de couplage ³*J*(NH, ^{β}CH) en Hz pour les résidus 2 à 7 de OL-7 en fonction de la température

| **température (K)** | **résidus** | | | | | |
|---|---|---|---|---|---|---|
| | **2** | **3** | **4** | **5** | **6** | **7** |
| 368 | - | 10,1 | 10,3 | 10,1 | 10,3 | 8,5 |
| 360 | - | 10,3 | 10,3 | 10,3 | 10,5 | 8,9 |
| 346 | - | 10,1 | 10,3 | 10,3 | 10,3 | 9,1 |
| 332 | 9,9 | 10,3 | 10,3 | 10,5 | 10,3 | 9,3 |
| 322 | - | 10,7 | 10,5 | 10,5 | 10,5 | 9,3 |
| 312 | - | 10,5 | 10,5 | 10,5 | 10,7 | - |
| 302 | 9,3 | 10,3 | 10,7 | 10,5 | 10,7 | 8,7 |
| 288 | 9,3 | 10,3 | 10,7 | 10,5 | 10,7 | 8,7 |

**Tableau 4.** Etude de la variation des constantes de couplage ³*J*(NH, ^{β}CH) en Hz pour les résidus 2 à 9 de OL-9 en fonction de la température

| **température (K)** | **résidus** | | | | | | | |
|---|---|---|---|---|---|---|---|---|
| | **2** | **3** | **4** | **5** | **6** | **7** | **8** | **9** |
| 308 | 9,5 | 10,1 | 11,3 | 10,4 | 10,1 | 10,1 | 10,7 | 7,9 |
| 332 | - | 9,8 | 10,4 | 9,8 | 10,4 | 10.4 | 10,1 | 8,9 |

Par ailleurs, les deux protons géminaux sur le ^{α}C présentent des différences significatives de déplacement chimique (1,3< Δδ<1,6 ppm pour les résidus centraux 3-6 et Δδ= 1,0-1,1 ppm pour les résidus 2 et 7). Cette différence qui reflète un arrangement spatial bien défini et distinct pour chacun des deux protons diastéréotopiques portés par le ^{α}C reste constante sur toute la gamme de température.

Dans le cas de l'oligomère OL-7, un examen minutieux des valeurs des constantes ³*J*(^{α}CH₂,N'H) et ³*J*(^{β}CH,^{α}CH₂) pour le résidu 3 à 332K (voir tableau 5 ci-après), révèle que le proton ^{α}CH le moins déblindé possède une constante de couplage élevée avec N'H (10Hz) et faible avec ^{β}CH (2,5Hz), ce qui implique que ce proton est dans un arrangement pratiquement *antipériplanaire* par rapport à N'H et *synclinal* par rapport à ^{β}CH.

**Tableau 5. Déplacements chimiques et constantes de couplage pour les protons du résidu 3 du composé OL-7.**

| **Proton** | **δ (ppm)** | **J (Hz)** |
|---|---|---|
| NH | 6,64 | ³J(NH,^{β}CH) = 10 |
| ^{β}CH | 4,02 | ³J(^{β}CH,^{α}CH^{Si}) = 2,5 ; ³J(^{β}CH,^{α}CH^{Re}) = 11 |
| ^{α}CH^{Re} | 2,53 | ²J (^{α}CH^{Re},^{α}CH^{Si})= 14 |
| ^{α}CH^{Si} | 3,96 | |
| N'H | 6,66 | ³J(N'H,^{α}CH^{Si}) = 10 ; ³J(N'H,^{α}CH^{Re}) = 3,5 |

Par la suite l'attribution stéréospécifique a été faite en supposant que les protons ^{α}CH avec une large constante de couplage ³*J*(^{α}CH₂,N'H) dans le spectre COSY sont les protons ^{α}CH*_{Si}*. Ces données indiquent que les oligomères OL-7 et OL-9 adoptent une structure secondaire stable et bien définie en solution.

Pour obtenir une information plus précise sur la structure secondaire, des spectres ROESY ont été enregistrés avec un temps de mélange de 300 ms. Un nombre important de corrélations NOE inter-résidus i/(i+2) peut être observé dans la pyridine pour OL-7 et OL-9 mais également dans le méthanol avec en particulier des corrélations intenses ^{β}CHᵢ/NHᵢ₊₂, ^{β}CHᵢ/N'Hᵢ₊₂ répétées tout au long de la séquence (voir tableau 6 ci-après).

Il est rappelé que les corrélations NOE correspondent à la mesure de l'effet "overhauser" entre deux protons (K. Wüthrich, NMR of proteins and nucleic acids, 1986, J. Wiley & Sons, Inc).

**Tableau 6. NOEs interrésidus pour OL-7 dans la pyridine-d₅ à 332K**

| atome H (résidu i) | atome H (résidu j) | NOE^{[a]} | k = j-i |
|---|---|---|---|
| N'H (1) | NH (2) | *s* | 1 |
| ^{α}H*_{Si}* (1) | NH (4) | *w* | 3 |
| ^{β}CH (1) | N'H (3) | *m* | 2 |
| N'H (2) | NH (3) | *s* | 1 |
| ^{β}CH (2) | N'H (3) | *m* | 1 |
| ^{β}CH (2) | NH (4) | *m* | 2 |
| ^{β}CH (2) | N'H (4) | *m* | 2 |
| N'H (3) | NH (4) | *s* | 1 |
| ^{α}H*_{Si}* (3) | N'H (5) | *s* | 2 |
| ^{β}CH (3) | NH (5) | *s* | 2 |
| ^{β}CH (3) | N'H (5) | *s* | 2 |
| ^{β}CH (3) | ^{α}H*_{Re}* (5) | *s* | 2 |
| ^{γ}CH (3) | ^{α}H*_{Re}* (5) | *w* | 2 |
| ^{δ}CH (3) | ^{α}H*_{Re}* (5) | *s* | 2 |
| N'H (4) | NH (5) | *s* | 1 |
| ^{β}CH (4) | NH (6) | *m* | 2 |
| ^{β}CH (4) | N'H (6) | *s* | 2 |
| N'H (5) | NH (6) | *s* | 1 |
| ^{α}H*_{Si}* (5) | NH (7) | *w* | 2 |
| ^{α}H*_{Si}* (5) | N'H (7) | *m* | 2 |
| ^{β}CH (5) | NH (7) | *s* | 2 |
| ^{β}CH (5) | N'H (7) | *m* | 2 |
| ^{β}CH (5) | ^{α}H*_{Re}* (7) | *m* | 2 |
| ^{γ}CH (5) | ^{α}H*_{Re}* (7) | *m* | 2 |
| N'H (6) | NH (7) | *s* | 1 |
| ^{β}CH (6) | N'H (7) | *w* | 1 |

| | | | |
|---|---|---|---|
| [a] fort (s), <2.8 Å ; médium (m), <3.8 Å ; faible (w), <5.5 Å. | | | |

Cette répétition des corrélations NOE tout au long de la séquence EST en accord avec une hélice de type 2.5-(P) déjà proposée dans le cas des γ-peptides. Nous rappelons qu'une hélice 2.5-(P) est une hélice droite (P) comportant 2,5 résidus par tour.

Les NOEs identifiés dans le spectre enregistré dans la pyridine ont été classés par catégorie de distances en fonction du volume des pics. Sur la base de ces contraintes de distance et des informations sur certains angles de torsion du squelette dérivés des constantes de couplage ³*J*, une modélisation moléculaire par recuit simulé et une étude par dynamique moléculaire ont été réalisées sur l'heptamère OL-7 en collaboration avec le groupe de Didier Rognan (UMR CNRS-ULP 7081, Strasbourg). Cette étude a confirmé la structuration en hélice de cet oligomère et la stabilité de la structure obtenue.

Il s'agit d'une hélice droite possédant un pas d'environ 5 angströms et un nombre de résidus par tour d'hélice d'environ 2,5. L'hélice est stabilisée tout au long de la séquence par un réseau de liaisons hydrogène intramoléculaires entre le C=O d'un résidu i et les deux NH (N'H et NH) d'une même fonction urée correspondant aux résidus i+2 et i+3 respectivement. La liaison hydrogène C=O*ᵢ* ----- NH_{*i*+3} (qui ferme un pseudocycle à 14 atomes) est statistiquement plus présente que la liaison hydrogène C=O*ᵢ* ----- N'H_{*i*+*2*} (qui ferme un pseudocycle à 12 atomes).

Sur la base de l'étude RMN dans le méthanol confirmant la structure dans ce solvant, une étude par dichroïsme circulaire (DC) a été effectuée dans le méthanol mais également dans l'eau et dans le trifluoroéthanol (voir Figure 3). Cette étude a révélé un signal caractéristique de la forme hélicoïdale (maximum à 205 nm) pour l'heptamère et pour le nonamère dans le méthanol et une absence de signal pour des oligomères plus courts (voir Figure 4) : un hexamère ne donne quasiment pas de signal à cette longueur d'onde. On peut donc conclure qu'un minimum de 7 liaisons urée est nécessaire à la formation de l'hélice dans le méthanol.

Dans le cas de l'oligomère OL-9, l'étude du signal à 205 nm en fonction de la température indique que celui ci est peu sensible à une augmentation de la température et suggère que la structure secondaire persiste jusqu'à 60°C et que le mécanisme de dépliement est non-coopératif (on obtient une droite de pente négative). Dans le trifluoroéthanol, le signal caractéristique disparaît alors que dans l'eau une partie du signal à 205 nm subsiste.

La présence d'un signal fort à 205 nm caractéristique de la structure secondaire en hélice suggère que le dichroïsme circulaire peut être utilisé avantageusement pour identifier rapidement la présence d'une structure secondaire dans des oligomères d'urée de séquence variée.

### TEST D'HEMOLYSE

### Principe du test :

Un composé agissant sur les membranes de bactéries peut potentiellement perturber les membranes de cellules de mammifère. Les globules rouges ou érythrocytes servent de modèle de cellules d'eucaryotes où le degré de la perméation membranaire est facilement évalué par mesure de la quantité d'hémoglobine libérée à travers les pores des membranes fragilisées et dont la concentration est directement proportionnelle à la densité optique à 450 nm.

### Utilisation du test :

Les érythrocytes sont préparés à partir de sang de singe fraîchement prélevé (macaque). Le sang héparinisé est centrifugé et lavé trois fois avec du PBS froid et remis en suspension à 10% (v/v) dans le PBS. A 90 µl de suspension de globules rouges sont rajoutés 10 µl d'une solution aqueuse des produits à tester. Les érythrocytes sont mis à incuber pendant une heure à 37°C. La suspension est centrifugée afin de récupérer le surnageant qui est plus ou moins rouge suivant le degré d'hémolyse. La concentration d'hémoglobine libérée dans le surnageant est mesurée par la densité optique à 450 nm. Dans le contrôle négatif les globules rouges sont incubés en absence de produit et montrent une DO de 0. L'hémolyse 100% est obtenue avec 100 µg de chlorure de cétylpyridinium par ml de suspension. Le degré d'hémolyse est évalué par rapport au témoin positif pour les différentes concentrations des produits testés.

### Résultats :

Les résultats du test d'hémolyse sont résumés dans le graphique de la Figure 5.

On constate que pour une concentration de 1 mg/ml, OL-9 conduit à 10% d'hémolyse alors que les peptides contrôle L et D avec les mêmes chaînes latérales conduisent respectivement, à la même concentration, à environ 50% et à plus de 60% d'hémolyse. On peut donc en conclure que les peptides naturels sont relativement toxiques pour les cellules eucaryotes alors que l'oligomère OL-9, qui démontre une activité antimicrobienne, est peu ou pas toxique pour les cellules eucaryotes à des doses de l'ordre de 1 mg/ml.

### TEST D'EVALUATION DE L'ACTIVITE ANTIMICROBIENNE ET/OU ANTIFONGIQUE

### Principe du test :

Les composés testés font tous partie de la classe polyurées et ont été obtenus au sein du laboratoire par voie chimique. Des tests d'inhibition de croissance de bactéries et de champignons ont été réalisés dans des tests liquides. L'activité spécifique de chaque molécule est exprimée par la concentration minimale inhibitrice de croissance des bactéries et/ou champignons, notée CMI. La CMI est une valeur spécifique pour chaque composé et varie en fonction des organismes testés. Il est à noter que les valeurs de la CMI suivent une progression logarithmique, ce qui est dû à une dilution deux à deux des composés testés dans une série de tests. Les molécules testées ont été présentées sous forme de sels solubles dans l'eau et ainsi le milieu de culture des microorganismes.

### Utilisation du test :

Pour la réalisation des tests, des plaques à 96 puits ont été utilisées. Chaque puits contient 90 µl d'une suspension de bactéries diluées dans du milieu PB (Poor Broth, Difco) pour avoir une concentration finale de 1 mDO. 10 µl du composé en solution dans de l'eau stérile à des concentrations passant de 1 mg/ml à 2 µg/ml sont rajoutés aux tests. Les plaques sont placées sous agitation à 29°C pendant 24 heures. La croissance des bactéries est mesurée par mesure de la turbidité du milieu de culture par mesure de la DO à 595 nm. La valeur de la CMI correspond à la concentration des substances à tester où aucune pousse de bactéries n'est constatée (DO = 0).

Les tests antifongiques sont réalisés de façon similaire avec du ½ PDB (Potato dextrose broth, Difco) comme milieu de culture et 10000 spores par ml de culture. Un contrôle optique sous microscope à optique inverse est nécessaire dans le cas des champignons pour évaluer la formation des mycéliums des champignons filamenteux (*Aspergillus fumigatus, Neurospora crassa*). La CMC est déterminée après 48 heures de culture à 29°C et correspond à la concentration où aucune formation de mycélium n'est observée.

Des contrôles internes ont été utilisés. Il s'agit du chlorure de cétylpyridinium et du bromure de déqualinium, antiseptiques classiques (Merck index).

### Résultats :

### Oligomère 9 (OL-9)

M.W. (+ 3 TFA) = 1732,82 g.mol⁻¹

### peptide L (contrôle)

M.W. (+ 3 TFA) = 1472,44 g.mol⁻¹

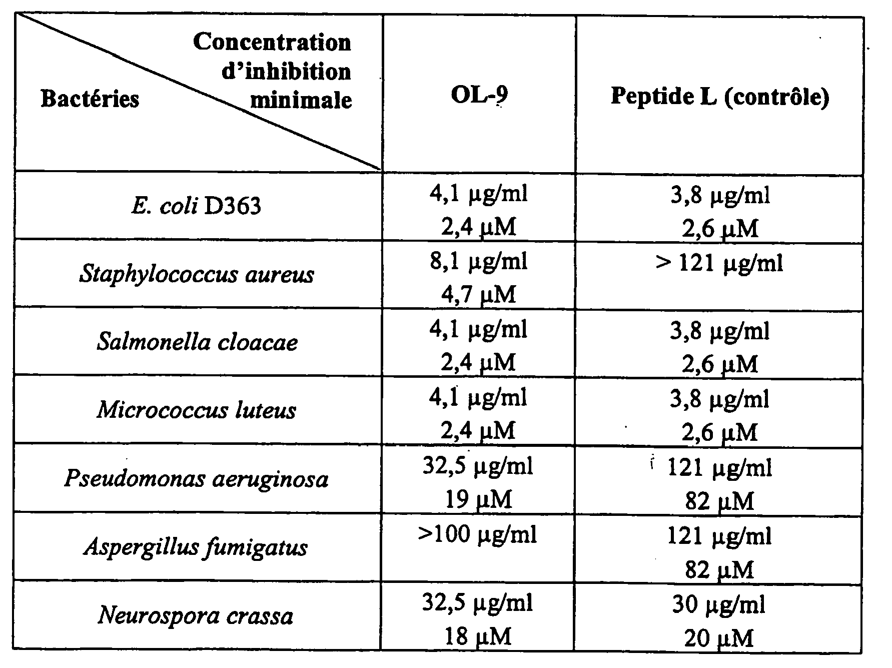

Les concentrations des composés testés dans une série de tests varient de 100 µg/ml de culture à 0,2 µg/ml. Des activités comprises entre des valeurs de 10 à 50 µg/ml sont considérées comme faibles. Des composés avec des activités entre 0,1 et 10 µg ont des activités du même ordre de grandeur que des antibiotiques commerciaux. Les meilleurs des composés testés ont une activité de 3 µg/ml avec un large spectre d'activités.

Il résulte de cette étude que l'oligomère OL-9 possède un spectre large d'activité antibactérienne *(E Coli, Staphylococcus aureus, Salmonella cloacae, Pseudomonas aeruginosa*) et que cette famille d'oligomères non naturels et leurs analogues oxygénés (oligocarbamates) représentent des candidats intéressants vers l'élaboration de nouveaux antibiotiques

### TEST D'EVALUATION DE LA CAPACITE D'INTERNALISATION DES OLIGOMERES

Les oligourées sont dissous dans du tampon phosphate (PBS, pH = 7.2), leur concentration est déterminée par absorption de la fluorescéine à 490 nm. La pesée de l'échantillon suivi de sa dissolution dans un volume connu de tampon PBS permet d'établir avec précision la concentration du transporteur. Les concentrations déterminées par spectroscopie UV sont cohérentes avec les quantités mesurées manuellement. La lignée de cellules T Jurkat, cultivée dans 10 µl de sérum de veau foetal en milieu Eagle modifié par Dulbecco (DMEM) a été utilisée pour toutes les mesures d'internalisation cellulaire. Des quantités variables d'oligomères sont ajoutées à 3 x 10⁵ cellules dans un volume total de 200 µl dans des puits de plaques de microtitration et sont incubées pendant 3 min. à 23°C. Les plaques sont centrifugées, les cellules sont isolées et lavées 3 fois avec du PBS froid puis resuspendue dans du PBS contenant 0,1% d'iodure de propidium. Les cellules sont alors analysées par cytométrie en flux à fluorescence (Faxscan, Becton Dickinson) et les cellules marquées à l'iodure de propidium exclues de l'analyse.

## Revendications

1. Utilisation des composés de formule générale :
X-(A)ₙ-Y, (I)
dans laquelle :
- n varie de 6 à 20,
- X représente un atome d'hydrogène, un groupe RₐCO, RₐOCO, RₐNHCO RₐSO₂ ou RₐNHCS,
Rₐ étant la fluorescéine ou un groupe alkyle (C1-C10), alkényle (C1-C10), alkynyle (C1-C10), aryle (C5-C12), aralkyle (C5-C14), hétéroaryle (C1-C5), lesdits groupes pouvant être non substitués ou substitués par 1 jusqu'à 6 substituants choisis parmi : un atome d'halogène, un groupe NO₂, OH, alkyle (C1-C4), NH₂, CN, trihalométhyle, acyloxy (C1-C4), dialkylamino (C1-C4), guanidino, un groupe SH, un groupe maléimide,
sous réserve que X soit différent de H lorsque n est égal à 6,
- A représente indifféremment : un groupe 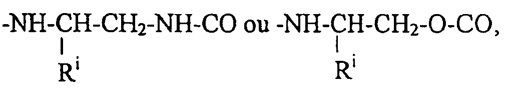
* R' étant un atome d'hydrogène, une chaîne latérale d'acide aminé, un groupe alkyle (C1-C10), alkényle (C1-C10), alkynyle (C1-C10), aryle (C5-C12), aralkyle (C5-C14) ou hétéroaryle (C1-C10), lesdits groupes pouvant être non substitués ou substitués par 1 jusqu'à 6 substituants choisis parmi : un atome d'halogène, un groupe NO₂, OH, amidine, benzamidine, imidazole, alkoxy, alkyle (C1-C4), NH₂, CN, trihalométhyle, acyloxy (C1-C4), dialkylamino (C1-C4), guanidino,
* i étant un nombre entier compris de 1 à n,
- Y est un groupe NR_{b}R_{c}, R_{b} et R_{c} ayant la signification donnée précédemment pour Rⁱ,
pour la préparation de médicaments destinés au traitement de maladies bactériennes, fongiques ou cytotoxiques, et notamment d'infections fongiques telles que les aspergilloses et les candidoses, et d'infections bactériennes résistantes.

2. Composés de formule générale :
X-(A)ₙ-Y, (I)
dans laquelle :
- n varie de 6 à 20,
- X représente un atome d'hydrogène, un groupe RₐCO, RₐOCO, RₐNHCO, RₐSO₂ ou RₐNHCS,
Rₐ étant la fluorescéine ou un groupe alkyle (C1-C10), alkényle (C1-C10), alkynyle (C1-C10), aryle (C5-C12), aralkyle (C5-C14), hétéroaryle (C1-C5), lesdits groupes pouvant être non substitués ou substitués par 1 jusqu'à 6 substituants choisis parmi : un atome d'halogène, un groupe NO₂, OH, alkyle (C1-C4), NH₂, CN, trihalométhyle, acyloxy (C1-C4), dialkylamino (C1-C4), guanidino, un groupe SH, un groupe maléimide,
sous réserve que X soit différent de H lorsque n est égal à 6,
- A représente indifféremment : un groupe 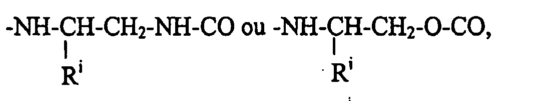
* R' étant un atome d'hydrogène, une chaîne latérale d'acide aminé, un groupe alkyle (C1-C10), alkényle (C1-C10), alkynyle (C1-C10), aryle (C5-C12), aralkyle (C5-C14) ou hétéroaryle (C1-C10), lesdits groupes pouvant être non substitués ou substitués par 1 jusqu'à 6 substituants choisis parmi : un atome d'halogène, un groupe NO₂, OH, amidine, benzamidine, imidazole, alkoxy, alkyle (C1-C4), NH₂, CN, trihalométhyle, acyloxy (C1-C4), dialkylamino (C1-C4), guanidino,
* i étant un nombre entier compris de 1 à n,
- Y est un groupe NR_{b}R_{c}, R_{b} et R_{c} ayant la signification donnée précédemment pour R',
sous réserve que les composés répondant aux formules suivantes soient exclus :

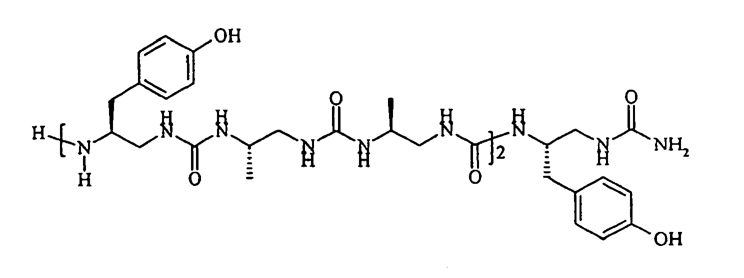

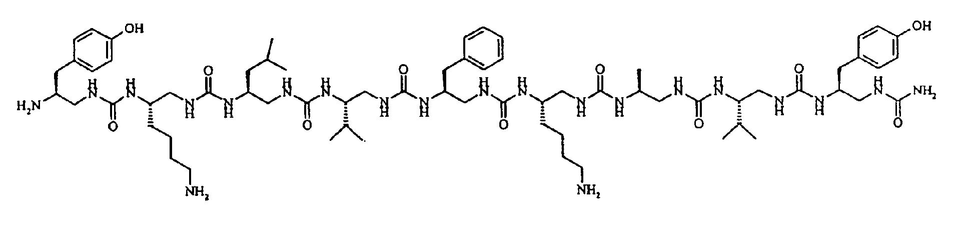

3. Composés selon la revendication 2, **caractérisés en ce que** n est égal à 6, 7, 8 ou 9, et répondant aux formules suivantes :
X-A³-A²-A³-A²-A²-A³-Y (13)
X-A²-A³-A²-A²-A³-A²-Y (14)
X-A³-A²-A³-A³-A²-A³-A²-Y (15)
X-A³-A²-A³-A²-A²-A³-A²-Y (16)
X-A²-A³-A²-A²-A³-A²-A³-Y (17)
X-A²-A³-A²-A³-A³-A²-A³-Y (18)
X-A³-A²-A³-A²-A²-A³-A²-A³-Y (19)
X-A²-A³-A²-A³-A³-A²-A³-A²-Y (20)
X-A¹-A²-A³-A³-A¹-A²-A³-A¹-A³-Y (1)
X-A¹-A²-A³-A³-A¹-A²-A³-A³-A¹-Y (2)
X-A¹-A²-A³-A³-A¹-A³-A²-A¹-A³-Y (3)
X-A¹-A²-A³-A³-A²-A¹-A³-A¹-A³-Y (4)
X-A¹-A²-A³-A²-A¹-A³-A³-A¹-A³-Y (5)
X-A¹-A²-A³-A³-A²-A¹-A³-A²-A³-Y (6)
X-A¹-A²-A³-A¹-A²-A³-A¹-A²-A³-Y (7)
X-A²-A¹-A³-A²-A¹-A³-A²-A¹-A³-Y (8)
X-A¹-A²-A³-A³-A²-A³-A³-A²-A¹-Y (9)
X-A¹-A³-A²-A³-A²-A³-A²-A³-A¹-Y (10)
X-A¹-A²-A³-A²-A¹-A²-A³-A²-A¹-Y (11)
X-A³-A²-A³-A²-A²-A³-A²-A³-A³-Y (12)
dans lesquelles :
- X et Y sont tels que définis dans la revendication 2,
- A¹ correspond au groupe A tel que défini dans la revendication 2, dans lequel R' représente une chaîne latérale aromatique, telle qu'un groupe benzyle correspondant à la chaîne latérale de la phénylalanine, 4-hydroxybenzyle correspondant à la chaîne latérale de la tyrosine ou (3-indolyl)méthyl correspondant à la chaîne latérale du tryptophane,
- A² correspond au groupe A tel que défini dans la revendication 2, dans lequel Rⁱ représente une chaîne basique correspondant à la lysine (-(CH₂)₄-NH₂), l'arginine (-(CH₂)₃NH-C(=NH)NH₂) ou l'omithinc (-(CH₂)₃-NH₂), ou un groupe alkyle (C1-C10), alkényle (C1-C10), alkynyle (C1-C10), lesdits groupes pouvant être non substitués ou substitués par 1 jusqu'à 6 substituants choisis parmi : un groupe amidine, NH₂, guanidino,
- A³ correspond au groupe A tel que défini dans la revendication 2, dans lequel Ri représente une chaîne latérale hydrophobe de type méthyle correspondant à la chaîne latérale de l'alanine, isopropyle correspondant à la chaîne latérale de la valine, isobutyle correspondant à la chaîne latérale de la leucine et sec-butyle correspondant à la chaîne latérale de l'isoleucine.

4. Composés selon la revendication 3, répondant aux formules suivantes : 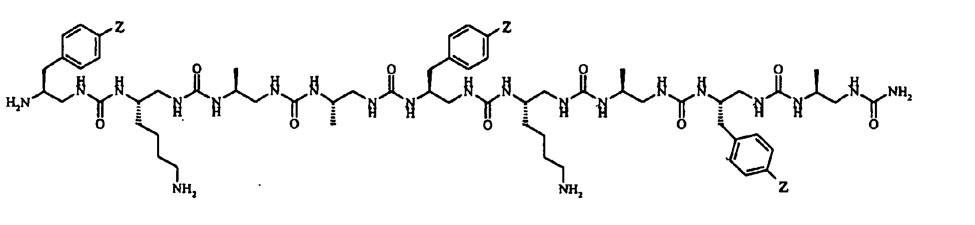 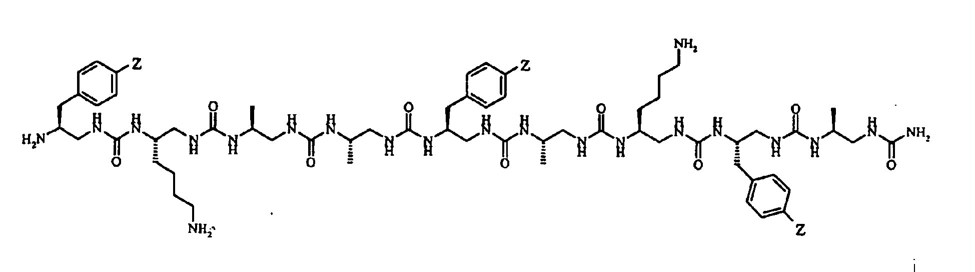 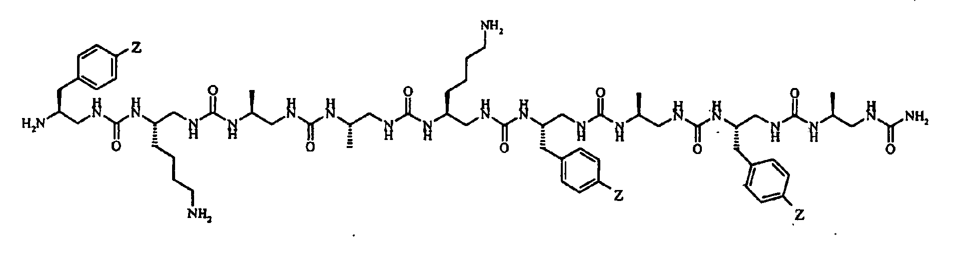 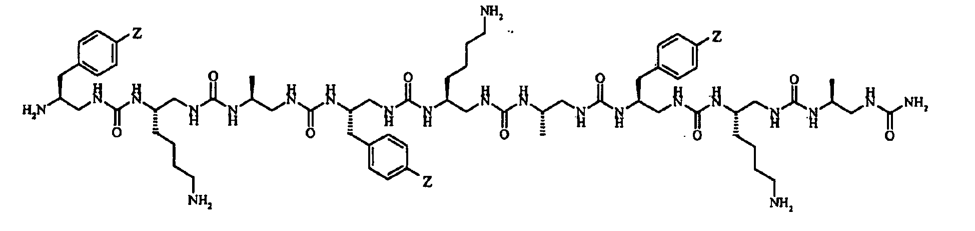 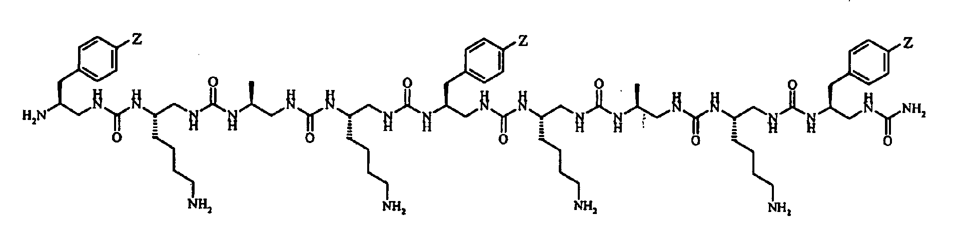 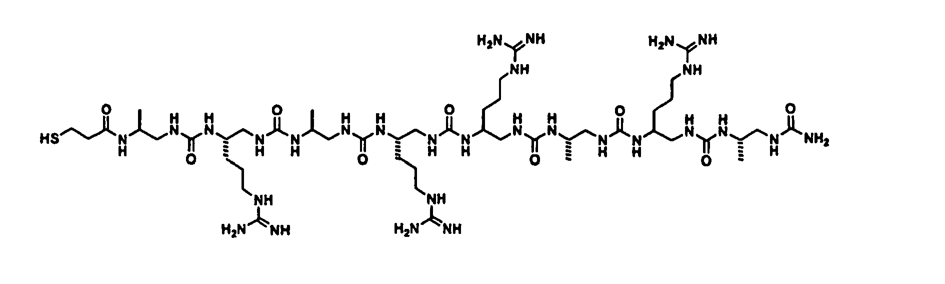 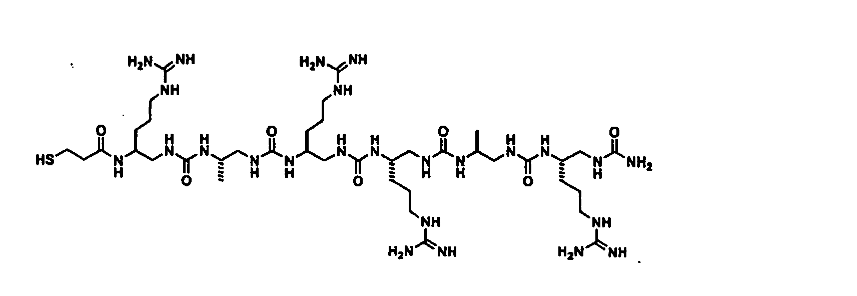 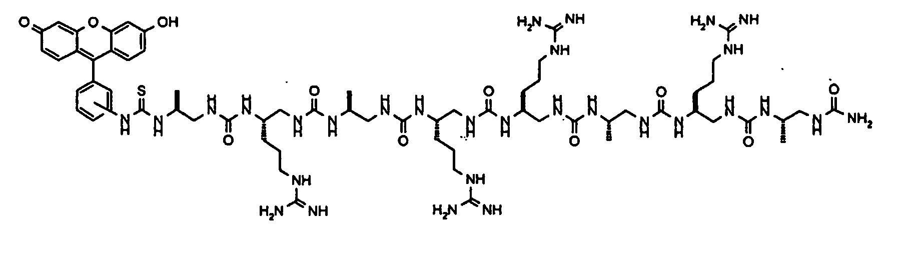 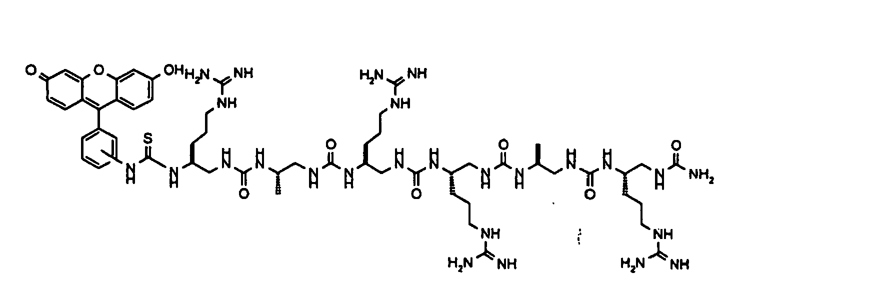 dans lesquelles Z représente soit un atome d'hydrogène soit un groupe OH.

5. Composés selon l'une des revendications 2 à 4, **caractérisés en ce qu'**au moins 10%, avantageusement de 10% à 50% des substituants Rⁱ sont des chaînes latérales d'acides aminés à caractère basique.

6. Composés selon la revendication 5, **caractérisés en ce que** les chaînes latérales d'acides aminés à caractère basique comportent des groupes amines primaire, secondaire ou tertiaire, des groupes imidazole, guanidine, amidine ou benzamidine.

7. Composés possédant une structure en hélice et répondant à la formule générale suivante :
X-(A)ₙ-Y, (I)
dans laquelle :
- n varie de 6 à 20,
- X représente un atome d'hydrogène, un groupe RₐCO, RₐOCO, RₐNHCO, RₐSO₂ ou RₐNHCS,
Rₐ étant un groupe alkyle (C1-C10), alkényle (C1-C10), alkynyle (C1-C10), aryle (C5-C12), aralkyle (C5-C14), hétéroaryle (C1-C5), lesdits groupes pouvant être non substitués ou substitués par 1 jusqu'à 6 substituants choisis parmi : un atome d'halogène, un groupe NO₂, OH, alkyle (C1-C4), NH₂, CN, trihalométhyle, acyloxy (C1-C4), dialkylamino (C1-C4), guanidino, un groupe SH, un groupe maléimide, la fluorescéine,
sous réserve que X soit différent de H lorsque n est égal à 6,
- A représente indifféremment : un groupe 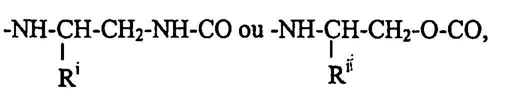
* R' étant un atome d'hydrogène, une chaîne latérale d'acide aminé, un groupe alkyle (C1-C10), alkényle (C1-C10), alkynyle (C1-C10), aryle (C5-C12), aralkyle (C5-C14) ou hétéroaryle (C1-C10), lesdits groupes pouvant être non substitués ou substitués par 1 jusqu'à 6 substituants choisis parmi : un atome d'halogène, un groupe NO₂, OH, amidine, benzamidine, imidazole, alkoxy, alkyle (C1-C4), NH₂, CN, trihalométhyle, acyloxy (C1-C4), dialkylamino (C1-C4), guanidino,
* i étant un nombre entier compris de 1 à n,
- Y est un groupe NR_{b}R_{c}, R_{b} et R_{c} ayant la signification donnée précédemment pour R',
ladite hélice présentant les caractéristiques suivantes :
- elle est de pas régulier à droite, compris de 4,9Å à 5,3Å, et notamment égal à 5Å,
- elle comprend de 2,4 à 2,6 résidus par tour et notamment 2,5 résidus par tour,
- son diamètre intérieur calculé à partir des centres des atomes est compris de 3,8Å à 4,6 Å, et notamment égal à 4,2Å,
- son diamètre intérieur calculé à partir de la surface de Van der Waals est compris de 1,4Å à 1,8 Å, et notamment égal à 1,6Å,

8. Composé selon la revendication 7 répondant à la formule suivante :

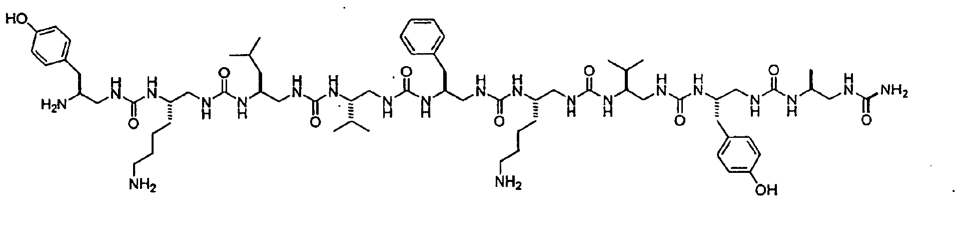

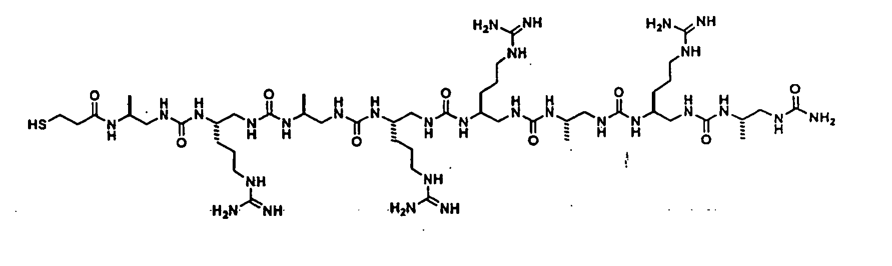

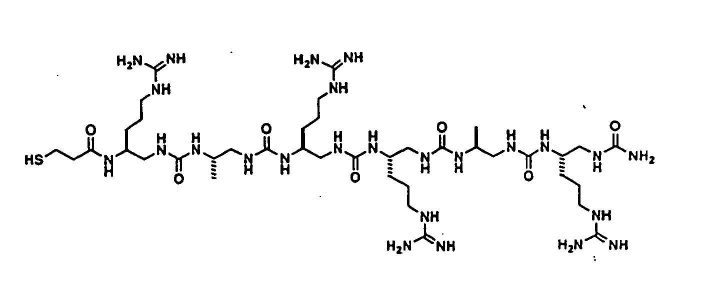

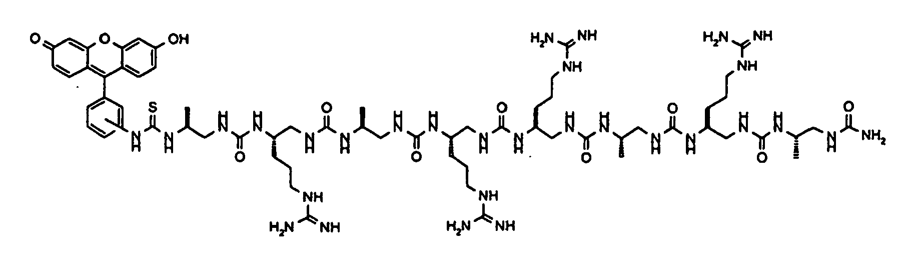

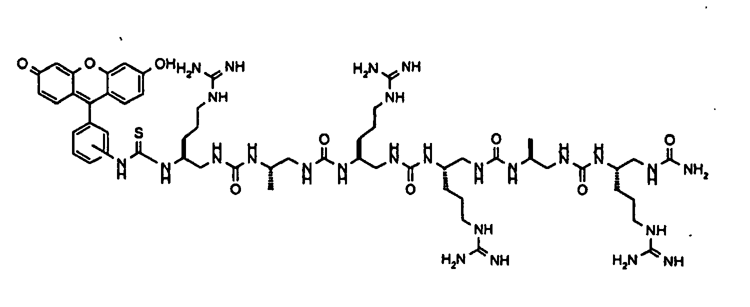

9. Composition contenant au moins un composé selon la revendication 7 et au moins un solvant, ledit solvant ayant la propriété de conférer aux molécules dudit composé une forme hélicoïdale, même partiellement, dans le cas d'un équilibre conformationnel entre les molécules dudit composé sous forme hélicoïdale et les molécules dudit composé sous forme non hélicoïdale, et étant notamment choisi parmi les alcools, la pyridine, l'acétonitrile, le diméthylsulfoxyde, l'eau en présence ou non de micelles ou de lipides, et étant avantageusement choisi parmi l'eau en présence ou non de micelles, le méthanol, l'éthanol, l'isopropanol, le trifluoroéthanol, l'hexafluoroisopropanol, le diméthylsulfoxyde ou tout mélange de ces solvants.

10. Composition pharmaceutique **caractérisée en ce qu'**elle comprend, à titre de substance active l'un au moins des composés selon l'une des revendications 2 à 6, en association avec un vecteur pharmaceutiquement acceptable.

11. Composition pharmaceutique selon la revendication 10, **caractérisée en ce qu'**elle contient par dose unitaire de 10 à 2000 mg de l'un des composés selon l'une des revendications 2 à 6.

12. Composition pharmaceutique **caractérisée en ce qu'**elle comprend le composé selon la revendication 8, en association avec un vecteur pharmaceutiquement acceptable.

13. Composition pharmaceutique **caractérisée en ce qu'**elle comprend, à titre de vecteur pharmaceutique l'un au moins des composés selon l'une des revendications 2 à 8 en association avec une substance pharmaceutiquement active.

14. Utilisation d'un composé selon l'une des revendications 2 à 8 comme vecteur pharmaceutique.

15. Procédé de préparation en phase solide des composés de formule (I) selon la revendication 2, à partir de monomères pré-activés de formule GP-A-W, dans laquelle :
- GP est un groupe protecteur choisi parmi Fmoc, Teoc, phtalimide, Alloc, BOC et Cbz,
- A est tel que défini dans la revendication 2,
- W est issu du groupe W-H choisi parmi : N-hydroxysuccinimide, phénol, pentafluorophénol, pentachlorophénol, p-nitrophénol, 2,4-dinitrophénol, 2,4,5-trichlorophénol, 2,4-dichloro-6-nitrophénol, hydroxy-1,2,3-benzotriazole, 1-oxo-2-hydroxydihydrobenzo-triazine (HODhbt), 7-aza-1-hydroxy-benzotriazole (HOAt), 4-aza-1-hydroxybenzo-triazole (4-HOAt), imidazole, tétrazole, et résine WANG, et est de préférence N-hydroxysuccinimide ou p-nitrophénol,
**caractérisé en ce qu'**il comprend les étapes suivantes :
a) une étape de couplage de GP-A-W tel que défini ci-dessus sur la fonction amine d'un support, ladite étape permettant le greffage du groupe -A-GP sur ladite fonction amine dudit support,
b) une étape de clivage du groupement GP dans des conditions appropriées,
c) la répétition séquentielle des étapes a) et b) jusqu'à ce que n groupes A aient été greffés sur ledit support,
d) une étape d'introduction des groupements RₐCO, RₐOCO, RₐNHCO ou RₐSO₂, Rₐ étant tel que défini dans la revendication 2,
e) une étape de clivage du composé de formule (I) du support par des moyens appropriés, qui permet le décrochage de la résine et la libération du groupe Y, tel que défini dans la revendication 2.

16. Procédé de préparation selon la revendication 15, **caractérisé en ce que** l'étape a) peut être effectuée en présence ou non d'une base tertiaire telle que DIEA, collidine, NMM ou lutidine, et en présence ou non d'un catalyseur tel que HOBt.

17. Procédé de préparation selon la revendication 15, **caractérisé en ce que** l'étape a) peut être effectuée dans un solvant tel que le DMF, CH₂Cl₂, THF ou N-méthylpyrrolidone.

18. Procédé de préparation selon la revendication 15, **caractérisé en ce que** le groupe GP est un groupe Fmoc.

19. Procédé de préparation en phase liquide des composés de formule (I) selon la revendication 2, à partir de monomères pré-activés de formule GP-A-W, dans laquelle :
- GP est un groupe protecteur choisi parmi Fmoc, Alloc, BOC et Cbz,
- A est tel que défini dans la revendication 2,
- W est issu du groupe W-H choisi parmi : N-hydroxysuccinimide, phénol, pentafluorophénol, pentachlorophénol, p-nitrophénol, 2,4-dinitrophénol, 2,4,5-trichlorophénol, 2,4-dichloro-6-nitrophénol, hydroxy-1,2,3-benzotriazole, 1-oxo-2-hydroxydihydrobenzo-triazine (HODhbt), 7-aza-1-hydroxy-benzotriazole (HOAt), 4-aza-1-hydroxybenzo-triazole (4-HOAt), imidazole et tétrazole, et est de préférence N-hydroxysuccinimide ou p-nitrophénol,
**caractérisé en ce qu'**il comprend les étapes suivantes :
a) une étape de couplage de GP-A-W tel que défini ci-dessus sur la fonction amine du groupe A du dérivé H-A-Y,
b) une étape de clivage du groupement GP dans des conditions appropriées,
c) la répétition séquentielle des étapes a) et b) jusqu'à ce que n groupes A aient été greffés sur la fonction amine mentionnée ci-dessus,
d) une étape d'introduction des groupements RₐCO, RₐOCO, RₐNHCO ou RₐSO₂, Rₐ étant tel que défini dans la revendication 2,
e) une étape de clivage du composé de formule (I) par des moyens appropriés.

20. Procédé de préparation des composés selon la revendication 7, par mise en contact d'un des composés définis dans la revendication 2 ou d'un des composés de formule (VIIc) ou (VIId), avec un solvant tel que le méthanol, l'éthanol, le trifluoroéthanol, l'hexafluoroisopropanol, l'eau, ou avec des tampons biologiques, tels que des tampons phosphate salins ou non, ou avec un milieu micellaire ou lipidique.

## Claims

1. Use of the compounds of general formula:
X-(A)ₙ-Y, (I)
in which:
- n varies from 6 to 20,
- X represents a hydrogen atom, an RₐCO, RₐOCO, RₐNHCO, RₐSO₂ or RₐNHCS group,
Rₐ being fluorescein or a (C1-C10) alkyl, (C1-C10) alkenyl, (C1-C10) alkynyl, (C5-C12) aryl, (C5-C14) aralkyl, (C1-C5) heteroaryl group, said groups being able to be non-substituted or substituted by 1 to 6 substituents chosen from: a halogen atom, an NO₂, OH, (C1-C4) alkyl, NH₂, CN, trihalomethyl, (C1-C4) acyloxy, (C1-C4) dialkylamino, guanidino group, an SH group, a maleimide group,
providing that X is different from H when n is equal to 6,
- A represents either:
an 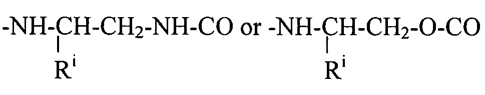 group,
* Rⁱ being a hydrogen atom, an amino acid side chain, a (C1-C10) alkyl, (C1-C10) alkenyl, (C1-C10) alkynyl, (C5-C12) aryl, (C5-C14) aralkyl or (C1-C10) heteroaryl group, said groups being able to be non-substituted or substituted by 1 to 6 substituents chosen from: a halogen atom, an NO₂, OH, amidine, benzamidine, imidazole, alkoxy, (C1-C4) alkyl, NH₂, CN, trihalomethyl, (C 1-C4) acyloxy, (C1-C4) dialkylamino, guanidino group,
* i being an integer comprised from 1 to n,
- Y is an NR_{b}R_{c} group, R_{b} and R_{c} having the meaning given previously for Rⁱ,
for the preparation of medicaments intended for the treatment of bacterial, fungal or cytotoxic diseases, and in particular fungal infections such as aspergillosis and candidosis, and resistant bacterial infections.

2. Compounds of general formula:
X-(A)ₙ-Y, (**I**)
in which:
- n varies from 6 to 20,
- X represents a hydrogen atom, an RₐCO, RₐOCO, RₐNHCO, RₐSO₂ or RₐNHCS group,
Rₐ being fluorescein or a (C1-C10) alkyl, (C1-C10) alkenyl, (C1-C10) alkynyl, (C5-C12) aryl, (C5-C14) aralkyl, (C1-C5) heteroaryl group, said groups being able to be non-substituted or substituted by 1 to 6 substituents chosen from: a halogen atom, an NO₂, OH, (C1-C4) alkyl, NH₂, CN, trihalomethyl, (C1-C4) acyloxy, (C1-C4) dialkylamino, guanidino group, an SH group, a maleimide group,
providing that X is different from H when n is equal to 6,
- A represents either: an 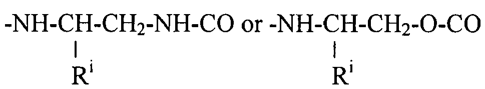 group,
* Rⁱ being a hydrogen atom, an amino acid side chain, a (C1-C10) alkyl, (C1-C10) alkenyl, (C1-C10) alkynyl, (C5-C12) aryl, (C5-C14) aralkyl or (C1-C10) heteroaryl group, said groups being able to be non-substituted or substituted by 1 to 6 substituents chosen from: a halogen atom, an NO₂, OH, amidine, benzamidine, imidazole, alkoxy, (C1-C4) alkyl, NH₂, CN, trihalomethyl, (C1-C4) acyloxy, (C1-C4) dialkylamino, guanidino group,
* i being an integer comprised from 1 to n,
- Y is a NR_{b}R_{c} group, R_{b} and R_{c} having the meaning given previously for R',
providing that the compounds corresponding to the following formulae are excluded:

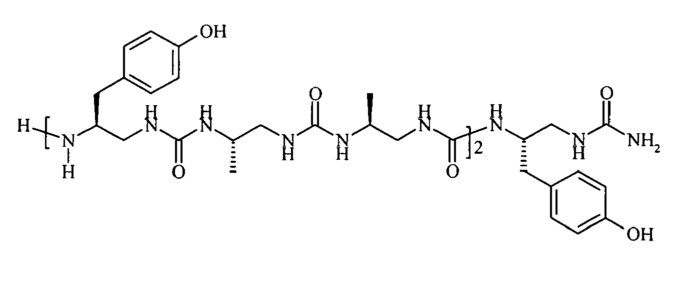

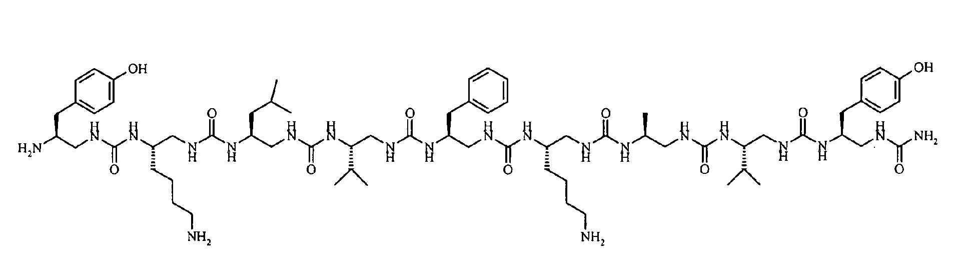

3. Compounds according to claim 2, **characterized in that** n is equal to 6, 7, 8 or 9, and corresponding to the following formulae:
X-A³-A²-A³-A²-A²-A³-Y (13)
X-A²-A³-A²-A²-A³-A²-Y (14)
X-A³-A²-A³-A³-A²-A³-A²-Y (15)
X-A³-A²-A³-A²-A²-A³-A²-Y (16)
X-A²-A³-A²-A²-A³-A²-A³-Y (17)
X-A²-A³-A²-A³-A³-A²-A³-Y (18)
X-A³-A²-A³-A²-A²-A³-A²-A³-Y (19)
X-A²-A³-A²-A³-A³-A²-A³-A²-Y (20)
X-A¹-A²-A³-A³-A¹-A²-A³-A¹-A³-Y (1)
X-A¹-A²-A³-A³-A¹-A²-A³-A³-A¹-Y (2)
X-A¹-A²-A³-A³-A¹-A³-A²-A¹-A³-Y (3)
X-A¹-A²-A³-A³-A²-A¹-A³-A¹-A³-Y (4)
X-A¹-A²-A³-A²-A¹-A³-A³-A¹-A³-Y (5)
X-A¹-A²-A³-A³-A²-A¹-A³-A²-A³-Y (6)
X-A¹-A²-A³-A¹-A²-A³-A¹-A²-A³-Y (7)
X-A²-A¹-A³-A²-A¹-A³-A²-A¹-A³-Y (8)
X-A¹-A²-A³-A³-A²-A³-A³-A²-A¹-Y (9)
X-A¹-A³-A²-A³-A²-A³-A²-A³-A¹-Y (10)
X-A¹-A²-A³-A²-A¹-A²-A³-A²-A¹-Y (11)
X-A³-A²-A³-A²-A²-A³-A²-A³-A³-Y (12)
in which:
- X and Y are as defined in claim 2,
- A¹ corresponds to the A group as defined in claim 2, in which Rⁱ represents an aromatic side chain, such as a benzyl group corresponding to the phenylalanine side chain, a 4-hydroxybenzyl group corresponding to the tyrosine side chain or a (3-indolyl)methyl group corresponding to the tryptophane side chain,
- A² corresponds to the A group as defined in claim 2, in which R' represents a basic chain corresponding to lysine (-(CH₂)₄-NH₂), arginine (-(CH₂)₃NH-C(=NH)NH₂) or ornithine (-(CH₂)₃-NH₂), or a (C1-C10) alkyl, (C1-C10) alkenyl, (C1-C10) alkynyl group, said groups being able to be non-substituted or substituted by 1 to 6 substituents chosen from: an amidine, NH₂, guanidino group,
- A³ corresponds to the group A as defined in claim 2, in which R' represents a hydrophobic side chain of methyl type corresponding to the alanine side chain, isopropyl corresponding to the valine side chain, isobutyl corresponding to the leucine side chain and sec-butyl corresponding to the isoleucine side chain.

4. Compounds according to claim 3, corresponding to the following formulae: 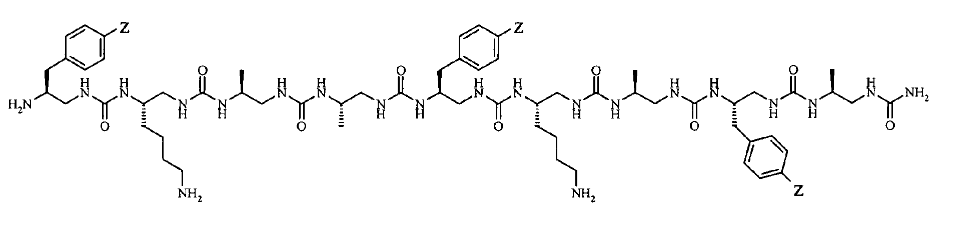 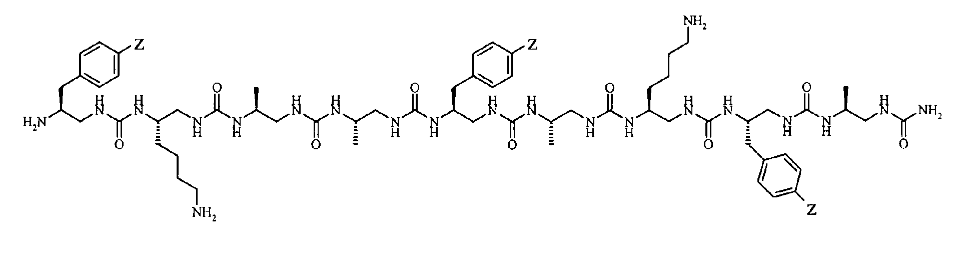 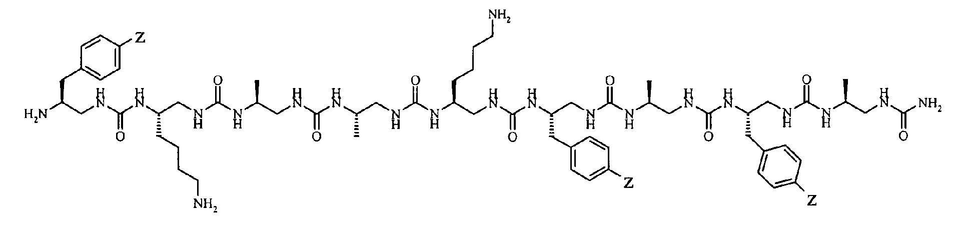 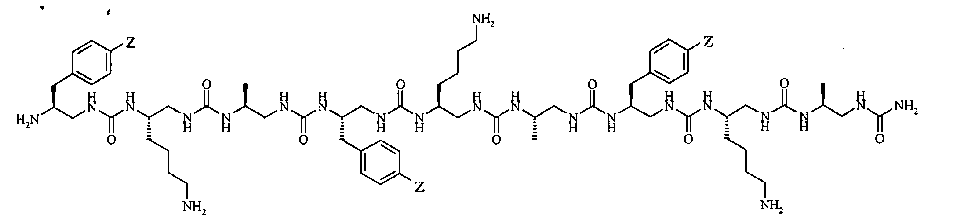 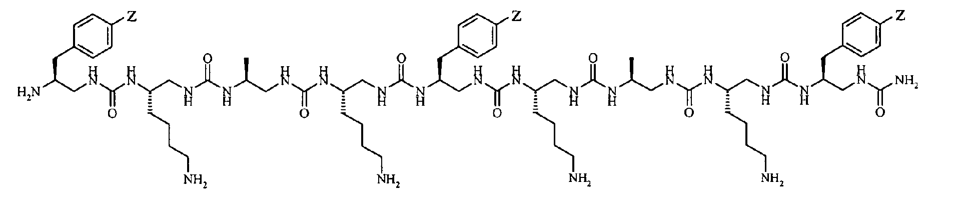 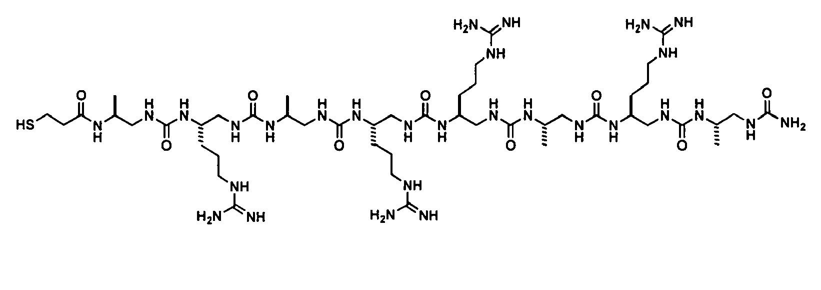 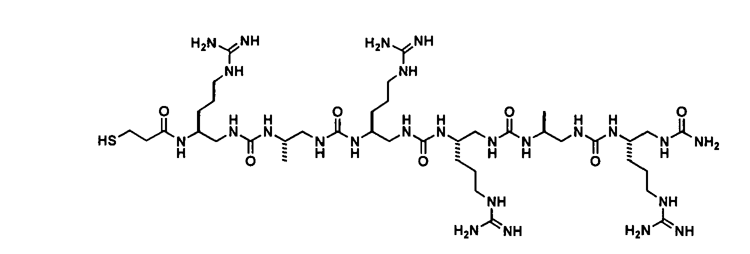 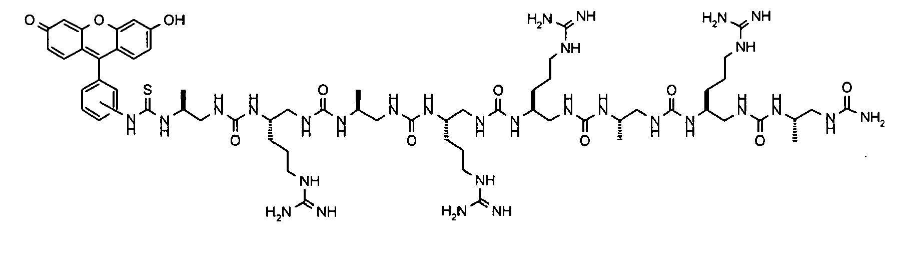 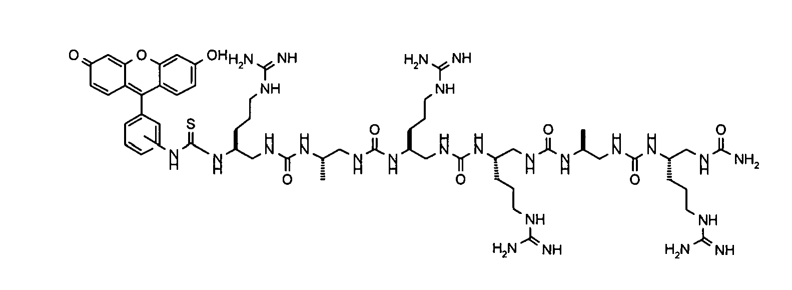 in which Z represents either a hydrogen atom or an OH group.

5. Compounds according to one of the claims 2 to 4, **characterized in that** at least 10%, advantageously 10% to 50% of the R' substituents are amino acid side chains of basic character.

6. Compounds according to claim 5, **characterized in that** the amino acid side chains of basic character comprise primary, secondary or tertiary amine groups, imidazole, guanidine, amidine or benzamidine groups.

7. Compounds possessing a helical structure and corresponding to the following general formula:
X-(A)ₙ-Y, (**I**)
in which:
- n varies from 6 to 20,
- X represents a hydrogen atom, a RₐCO, RₐOCO, RₐNHCO, RₐSO₂ or RₐNHCS group,
Rₐ being a (C1-C10) alkyl, (C1-C10) alkenyl, (C1-C10) alkynyl, (C5-C12) aryl, (C5-C14) aralkyl, (C1-C5) heteroaryl group, said groups being able to be non-substituted or substituted by 1 to 6 substituents chosen from: a halogen atom, an NO₂, OH, (C1-C4) alkyl, NH₂, CN, trihalomethyl, (C1-C4) acyloxy, (C1-C4) dialkylamino, guanidino group, an SH group, a maleimide group, fluorescein,
providing that X is different from H when n is equal to 6,
- A represents either:
a 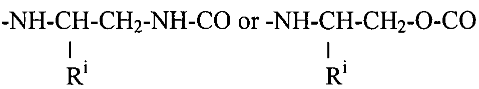 group,
* R' being a hydrogen atom, an amino acid side chain, a (C1-C10) alkyl, (C1-C10) alkenyl, (C1-C10) alkynyl, (C5-C12) aryl, (C5-C14) aralkyl or (C1-C10) heteroaryl group, said groups being able to be non-substituted or substituted by 1 to 6 substituents chosen from: a halogen atom, an NO₂, OH, amidine, benzamidine, imidazole, alkoxy, (C1-C4) alkyl, NH₂, CN, trihalomethyl, (C1-C4) acyloxy, (C1-C4) dialkylamino, guanidino group,
* i being an integer comprised from 1 to n,
- Y is a NR_{b}R_{c} group, R_{b} and R_{c} having the meaning given previously for Rⁱ, said helix having the following characteristics:
- it has a regular pitch to the right, comprised from 4.9 Å to 5.3 Å, and in particular equal to 5 Å,
- it comprises from 2.4 to 2.6 residues per turn and in particular 2.5 residues per turn,
- its internal diameter calculated from the centres of the atoms is comprised from 3.8 Å to 4.6 Å, and in particular equal to 4.2 Å,
- its internal diameter calculated from the Van der Waals surface is comprised from 1.4 Å to 1.8 Å, and in particular equal to 1.6 Å,

8. Compound according to claim 7 corresponding to the following formula:

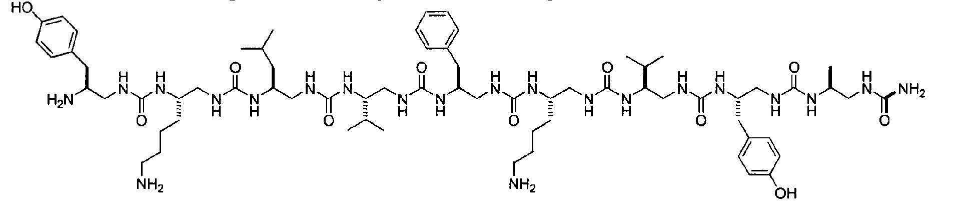

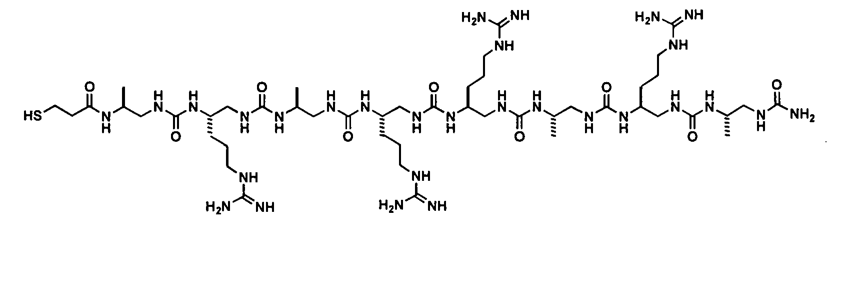

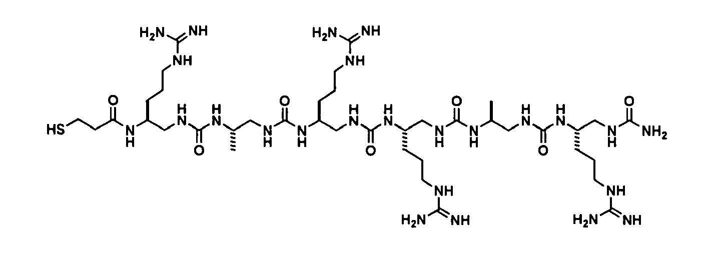

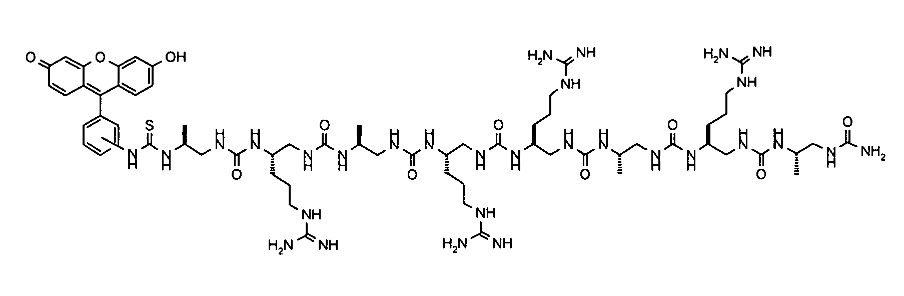

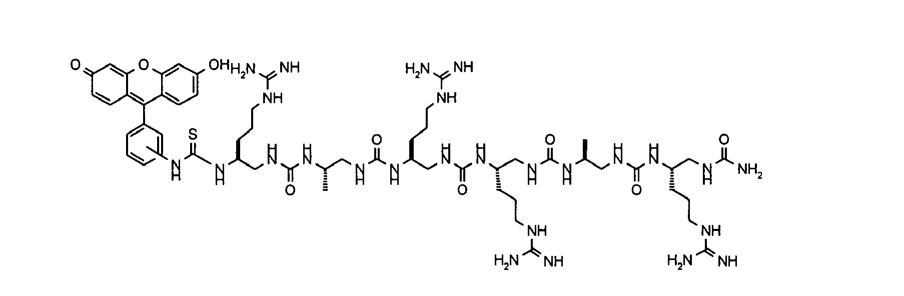

9. Composition containing at least one compound according to claim 7 and at least one solvent, said solvent having the property of conferring upon the molecules of said compound a helicoidal form, even partially, in the case of a conformational equilibrium between the molecules of said compound in the helicoidal form and the molecules of said compound in the non-helicoidal form, and being in particular chosen from alcohols, pyridine, acetonitrile, dimethylsulphoxide, water in the presence or absence of micelles or lipids, and being advantageously chosen from water in the presence or absence of micelles, methanol, ethanol, isopropanol, trifluoroethanol, hexafluoroisopropanol, dimethylsulphoxide or any mixture of these solvents.

10. Pharmaceutical composition **characterized in that** it comprises, as active ingredient at least one of the compounds according to one of claims 2 to 6, in combination with a pharmaceutically acceptable vector.

11. Pharmaceutical composition according to claim 10, **characterized in that** it contains per unit dose from 10 to 2000 mg of one of the compounds according to one of claims 2 to 6.

12. Pharmaceutical composition **characterized in that** it comprises the compound according to claim 8, in combination with a pharmaceutically acceptable vector.

13. Pharmaceutical composition **characterized in that** it comprises, as pharmaceutical vector at least one of the compounds according to one of claims 2 to 8 in combination with a pharmaceutically active ingredient.

14. Use of a compound according to one of claims 2 to 8 as pharmaceutical vector.

15. Process for the preparation in solid phase compounds of formula (I) according to claim 2, from pre-activated monomers of formula GP-A-W, in which:
- GP is a protective group chosen from Fmoc, Teoc, phtalimide, Alloc, BOC and Cbz,
- A is as defined in claim 2,
- W originates from the W-H group chosen from: N-hydroxysuccinimide, phenol, pentafluorophenol, pentachlorophenol, p-nitrophenol, 2,4-dinitrophenol, 2,4,5-trichlorophenol, 2,4-dichloro-6-nitrophenol, hydroxy-1,2,3-benzotriazole, 1-oxo-2-hydroxydihydrobenzotriazine (HODhbt), 7-aza-1-hydroxy-benzotriazole (HOAt), 4-aza-1-hydroxybenzo-triazole (4-HOAt), imidazole, tetrazole, and WANG resin, and is preferably N-hydroxysuccinimide or p-nitrophenol,
**characterized in that** it comprises the following stages:
a) a stage of coupling of GP-A-W as defined above on the amine function of a support, said stage allowing the grafting of the -A-GP group to said amine function of said support,
b) a cleavage stage of the GP group under appropriate conditions,
c) the sequential repetition of stages a) and b) until n A groups have been grafted to said support,
d) a stage of introduction of the RₐCO, RₐOCO, RₐNHCO or RₐSO₂ groups, Rₐ being as defined in claim 2,
e) a stage of cleavage of the compound of formula (I) from the support by appropriate means, which allows the removal of the resin and the release of the Y group, as defined in claim 2.

16. Preparation process according to claim 15, **characterized in that** stage a) can be carried out in the presence or absence of a tertiary base such as DIEA, collidine, NMM or lutidine, and in the presence or absence of a catalyst such as HOBt.

17. Preparation process according to claim 15, **characterized in that** stage a) can be carried out in a solvent such as DMF, CH₂Cl₂, THF or N-methylpyrrolidone.

18. Preparation process according to claim 15, **characterized in that** the GP group is an Fmoc group.

19. Process for the preparation in liquid phase of the compounds of formula (I) according to claim 2, from pre-activated monomers of formula GP-A-W, in which:
- GP is a protective group chosen from Fmoc, Alloc, BOC and Cbz,
- A is as defined in claim 2,
- W originates from the W-H group chosen from: N-hydroxysuccinimide, phenol, pentafluorophenol, pentachlorophenol, p-nitrophenol, 2,4-dinitrophenol, 2,4,5-trichlorophenol, 2,4-dichloro-6-nitrophenol, hydroxy-1,2,3-benzotriazole, 1-oxo-2-hydroxydihydrobenzotriazine (HODhbt), 7-aza-1-hydroxy-benzotriazole (HOAt), 4-aza-1-hydroxybenzo-triazole (4-HOAt), imidazole and tetrazole, and is preferably N-hydroxysuccinimide or p-nitrophenol,
**characterized in that** it comprises the following stages:
a) a stage of coupling of GP-A-W as defined above to the amine function of the A group of the H-A-Y derivative,
b) a stage of cleavage of the GP group under appropriate conditions,
c) the sequential repetition of stages a) and b) until n A groups have been grafted to the amine function mentioned above,
d) a stage of introduction of the RₐCO, RₐOCO, RₐNHCO or RₐSO₂ groups, Rₐ being as defined in claim 2,
e) a stage of cleavage of the compound of formula (I) by appropriate means.

20. Process for the preparation the compounds according to claim 7, by bringing one of the compounds as defined above or one of the compounds of formula (VIIc) or (VIId), into contact with a solvent such as methanol, ethanol, trifluoroethanol, hexafluoroisopropanol, water, or with biological buffers, such as saline or non-saline phosphate buffers, or with a micelle or lipid medium.

## Patentansprüche

1. Verwendung von Verbindungen mit der allgemeinen Formel:
X-(A)ₙ-Y, (I)
wobei:
- n zwischen 6 und 20 variiert,
- X für ein Wasserstoffatom, eine RₐCO-, RₐOCO-, RₐNHCO-, RₐSO₂- oder eine RₐNHCS-Gruppe steht,
wobei es sich bei Rₐ um Fluorescein oder um eine Alkyl(C1-C10)-, Alkenyl(C1-C10)-, Alkinyl(C1-C10)-, Aryl(C5-C12)-, Aralkyl(C5-C14) oder Heteroaryl(C1-C5)-Gruppe handelt, wobei diese Gruppen unsubstituiert sein können oder 1 bis maximal 6 Substituenten aufweisen können, die aus den folgenden gewählt sind: einem Halogenatom, einer NO₂-, OH-, Alkyl(C1-C4)-, NH₂-, CN-, Trihalogenmethyl-, Acyloxy(C1-C4)-, Dialkylamino(C1-C4)-, Guanidino- oder einer SH-Gruppe oder einer Maleimidgruppe,
mit der Maßgabe, dass X sich von H unterscheidet, wenn n gleich 6 ist,
- A wahlweise für Folgendes steht:
eine 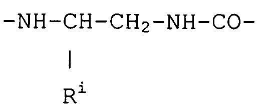 oder eine 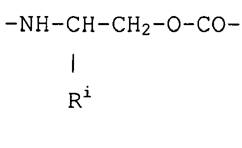 Gruppe,
* wobei es sich bei Rⁱ um ein Wasserstoffatom, eine Aminosäureseitenkette, eine Alkyl(C1-C10)-, Alkenyl(C1-C10)-, Alkinyl(C1-C10)-, Aryl(C5-C12)-, Aralkyl(C5-C12)- oder Heteroaryl(C1-C10)-Gruppe handelt, wobei diese Gruppen unsubstituiert sein können oder 1 bis maximal 6 Substituenten aufweisen können, die aus den folgenden gewählt sind: einem Halogenatom, einer NO₂-, OH-, Amidin-, Benzamidin-, Imidazol-, Alkoxy-, Alkyl(C1-C4)-, NH₂-, CN-, Trihalogenmethyl-, Acyloxy(C1-C4)-, Dialkylamino(C1-C4) - oder einer Guanidinogruppe,
* wobei es sich bei i um eine ganze Zahl von 1 bis n handelt,
- es sich bei Y um eine NR_{b}R_{c}-Gruppe handelt, wobei R_{b} und R_{c} die Bedeutung haben, die vorstehend für Rⁱ angegeben wurde,
zur Herstellung von Arzneimitteln, die zur Behandlung von bakteriellen, pilzbedingten oder zytotoxischen Krankheiten sowie insbesondere von Pilzinfektionen wie etwa Aspergillosen und Candidosen und von Infektionen mit resistenten Bakterien bestimmt sind.

2. Verbindungen mit der allgemeinen Formel:
X-(A)ₙ-Y, (I)
wobei:
- n zwischen 6 und 20 variiert,
- X für ein Wasserstoffatom, eine RₐCO-, RₐOCO-, RₐNHCO- RₐSO₂- oder eine RₐNHCS-Gruppe steht,
wobei es sich bei Rₐ um Fluorescein oder um eine Alkyl(C1-C10)-, Alkenyl(C1-C10)-, Alkinyl(C1-C10)-, Aryl(C5-C12)-, Aralkyl(C5-C14) oder Heteroaryl(C1-C5)-Gruppe handelt, wobei diese Gruppen unsubstituiert sein können oder 1 bis maximal 6 Substituenten aufweisen können, die aus den folgenden gewählt sind: einem Halogenatom, einer NO₂-, OH-, Alkyl(C1-C4)-, NH₂-, CN-, Trihalogenmethyl-, Acyloxy(C1-C4)-, Dialkylamino(C1-C4)-, Guanidino-, einer SH- oder einer Maleimidgruppe,
mit der Maßgabe, dass X sich von H unterscheidet, wenn n gleich 6 ist,
- A wahlweise für Folgendes steht:
eine 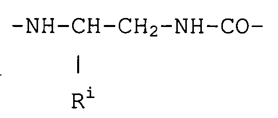 oder eine 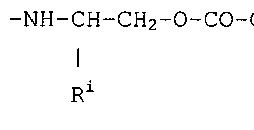 Gruppe,
* wobei es sich bei Rⁱ um ein Wasserstoffatom, eine Aminosäureseitenkette, eine Alkyl(C1-C10)-, Alkenyl(C1-C10)-, Alkinyl(C1-C10)-, Aryl(C5-C12)-, Aralkyl(C5-C12)- oder Heteroaryl(C1-C10)-Gruppe handelt, wobei diese Gruppen unsubstituiert sein können oder 1 bis maximal 6 Substituenten aufweisen können, die aus den folgenden gewählt sind: einem Halogenatom, einer NO₂-, OH-, Amidin-, Benzamidin-, Imidazol-, Alkoxy-, Alkyl(C1-C4)-, NH₂-, CN-, Trihalogenmethyl-, Acyloxy(C1-C4)-, Dialkylamino(C1-C4)- oder einer Guanidinogruppe,
* wobei es sich bei i um eine ganze Zahl von 1 bis n handelt,
- es sich bei Y um eine NR_{b}R_{c}-Gruppe handelt, wobei R_{b} und R_{c} die Bedeutung haben, die vorstehend für Rⁱ angegeben wurde,
mit der Maßgabe, dass die Verbindungen nach den folgenden Formeln ausgeschlossen sind:

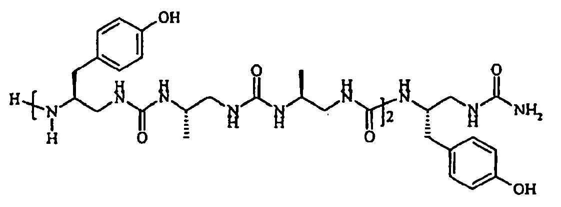

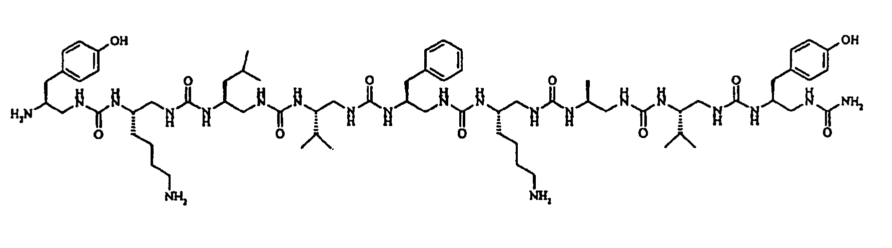

3. Verbindungen nach Anspruch 2, **dadurch gekennzeichnet, dass** n gleich 6, 7, 8 oder 9 ist und einer der folgenden Formeln entspricht:
X-A³-A²-A³-A²-A²-A³-Y (13)
X-A²-A³-A²-A²-A³-A²-Y (14)
X-A³-A²-A³-A³-A²-A³-A²-Y (15)
X-A³-A²-A³-A²-A²-A³-A²-Y (16)
X-A²-A³-A²-A²-A³-A²-A³-Y (17)
X-A²-A³-A²-A³-A³-A²-A³-Y (18)
X-A³-A²-A³-A²-A²-A³-A²-A³-Y (19)
X-A²-A³-A²-A³-A³-A²-A³-A²-Y (20)
X-A¹-A²-A³-A³-A¹-A²-A³-A¹-A³-Y (1)
X-A¹-A²-A³-A³-A¹-A²-A³-A³-A¹-Y (2)
X-A¹-A²-A³-A³-A¹-A³-A²-A¹-A³-Y (3)
X-A¹-A²-A³-A³-A²-A¹-A³-A¹-A³-Y (4)
X-A¹-A²-A³-A²-A¹-A³-A³-A¹-A³-Y (5)
X-A¹-A²-A³-A³-A²-A¹-A³-A²-A³-Y (6)
X-A¹-A²-A³-A¹-A²-A³-A¹-A²-A³-Y (7)
X-A²-A¹-A³-A²-A¹-A³-A²-A¹-A³-Y (8)
X-A¹-A²-A³-A³-A²-A³-A³-A²-A¹-Y (9)
X-A¹-A³-A²-A³-A²-A³-A²-A³-A¹-Y (10)
X-A¹-A²-A³-A²-A¹-A²-A³-A²-A¹-Y (11)
X-A³-A²-A³-A²-A²-A³-A²-A³-A³-A³-Y (12)
in welchen:
- X und Y den Begriffsbestimmungen in Anspruch 2 entsprechen,
- A¹ einer Gruppe A gemäß der Begriffsbestimmung in Anspruch 2 entspricht, wobei Rⁱ für eine aromatische Seitenkette steht, wie etwa für eine Benzylgruppe, welche der Seitenkette des Phenylalanins entspricht, für eine 4-Hydroxybenzylgruppe, welche der Seitenkette des Tyrosins entspricht, oder für eine (3-Indolyl)methylgruppe, welche der Seitenkette des Tryptophans entspricht,
- A² einer Gruppe A gemäß der Begriffsbestimmung in Anspruch 2 entspricht, wobei Rⁱ für eine basische Kette steht, welche dem Lysin (-(CH₂)₄-NH₂), dem Arginin (-(CH₂)₃NH-C(=NH)NH₂) oder dem Ornithin (-(CH₂)₃-NH₂) entspricht, oder für eine Alkyl(C1-C10)-, Alkenyl(C1-C10)-, Alkinyl(C1-C10)-Gruppe,
wobei diese Gruppen unsubstituiert sein können oder 1 bis maximal 6 Substituenten aufweisen können, die aus den folgenden gewählt sind: einer Amidin-, NH₂- oder Guanidinogruppe.
- A³ einer Gruppe A gemäß der Begriffsbestimmung in Anspruch 2 entspricht, wobei Rⁱ für eine hydrophobe Seitenkette des Typs Methyl steht, welche der Seitenkette des Alanin entspricht, des Typs Isopropyl, welche der Seitenkette des Valins entspricht, des Typs Isobutyl, welche der Seitenkette des Leucins entspricht, oder des Typs sec.-Butyl, welche der Seitenkette des Isoleucins entspricht.

4. Verbindungen nach Anspruch 3, welche den folgenden Formeln entsprechen: 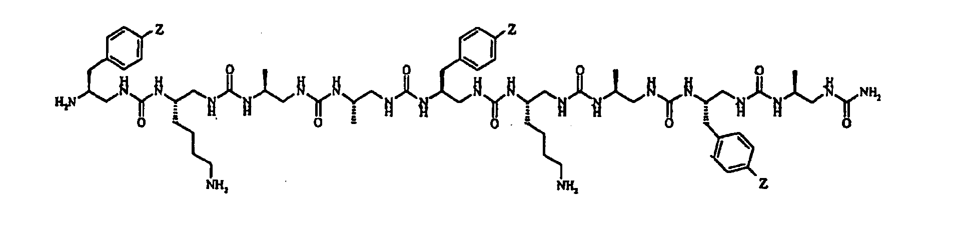 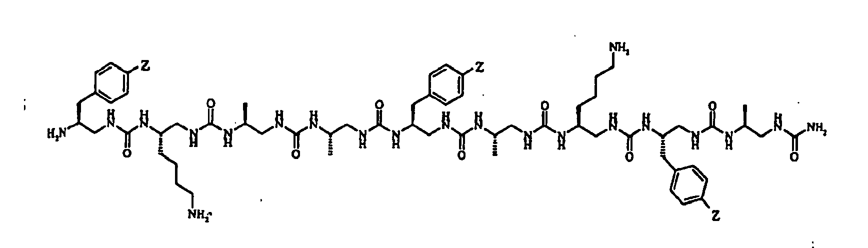 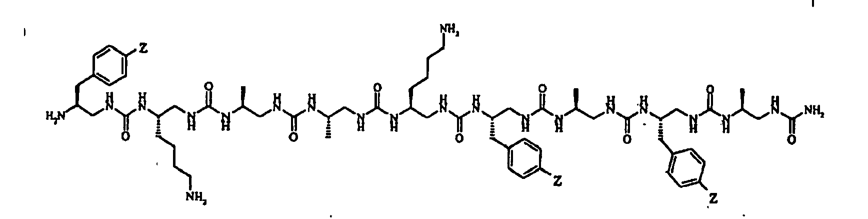 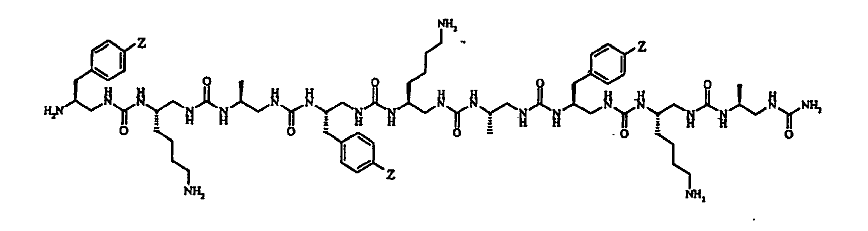 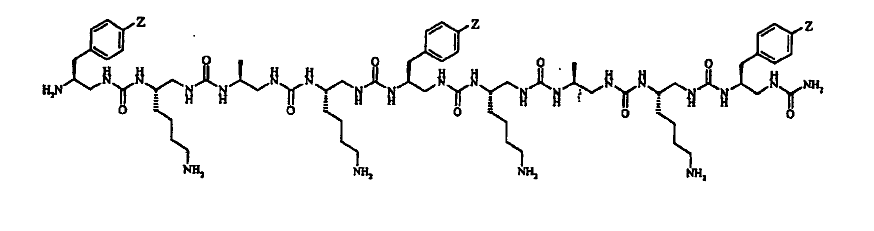 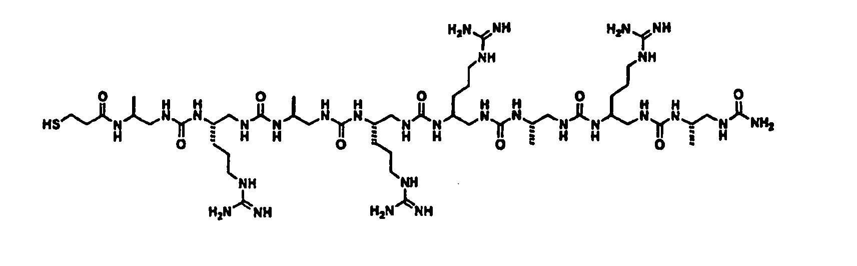 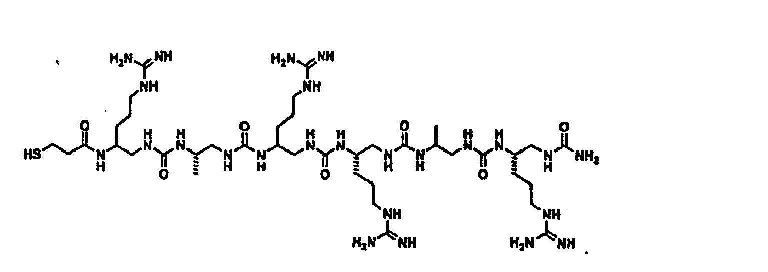 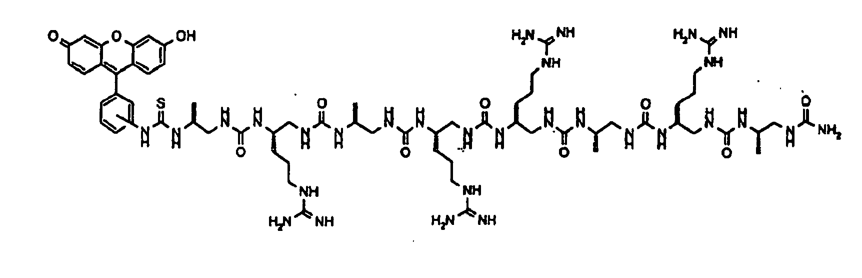 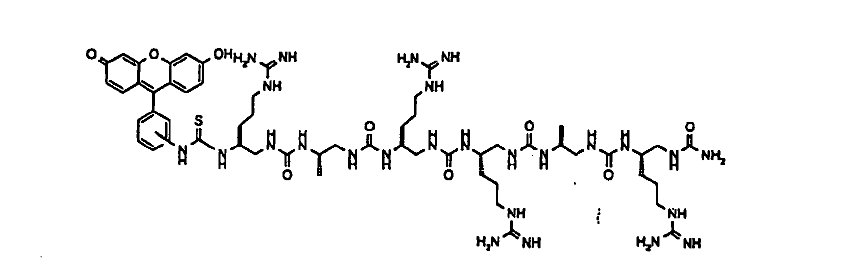 in welchen Z entweder für ein Wasserstoffatom oder für eine OH-Gruppe steht.

5. Verbindungen nach einem der Ansprüche 2 bis 4, **dadurch gekennzeichnet, dass** es sich bei mindestens 10 %, vorteilhafterweise bei 10 % bis 50 % der Substituenten Rⁱ um Seitenketten von basischen Aminosäuren handelt.

6. Verbindungen nach Anspruch 5, **dadurch gekennzeichnet, dass** die Seitenketten der basischen Aminosäuren primäre, sekundäre, oder tertiäre Aminogruppen, Imidazol-, Guanidin-, Amidin- oder Benzamidingruppen aufweisen.

7. Verbindungen, die eine Helixstruktur aufweisen und der folgenden allgemeinen Formel entsprechen:
X-(A)ₙ-Y, (I)
wobei:
- n zwischen 6 und 20 variiert,
- X für ein Wasserstoffatom, eine RₐCO-, RₐOCO-, RₐNHCO- RₐSO₂- oder eine RₐNHCS-Gruppe steht,
wobei es sich bei Rₐ um eine Alkyl(C1-C10)-, Alkenyl(C1-C10)-, Alkinyl(C1-C10)-, Aryl(C5-C12)-, Aralkyl(C5-C14) oder Heteroaryl(C1-C5)-Gruppe handelt, wobei diese Gruppen unsubstituiert sein können oder 1 bis maximal 6 Substituenten aufweisen können, die aus den folgenden gewählt sind: einem Halogenatom, einer NO₂-, OH-, Alkyl(C1-C4)-, NH₂-, CN-, Trihalogenmethyl-, Acyloxy(C1-C4)-, Dialkylamino(C1-C4)- Guanidino, einer SH-Gruppe, einer Maleimidgruppe, Fluorescein,
mit der Maßgabe, dass X sich von H unterscheidet, wenn n gleich 6 ist,
- A wahlweise für Folgendes steht:
eine 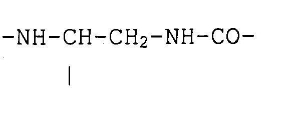 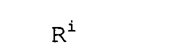 oder eine 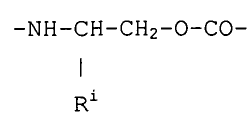 Gruppe,
* wobei es sich bei Rⁱ um ein Wasserstoffatom, eine Aminosäureseitenkette, eine Alkyl(C1-C10)-, Alkenyl(C1-C10)-, Alkinyl(C1-C10)-, Aryl(C5-C12)-, Aralkyl(C5-C14)- oder Heteroaryl(C1-C10)-Gruppe handelt, wobei diese Gruppen unsubstituiert sein können oder 1 bis maximal 6 Substituenten aufweisen können, die aus den folgenden gewählt sind: einem Halogenatom, einer NO₂-, OH-, Amidin-, Benzamidin-, Imidazol-, Alkoxy-, Alkyl(C1-C4)-, NH₂-, CN-, Trihalogenmethyl-, Acyloxy(C1-C4)-, Dialkylamino(C1-C4)- oder einer Guanidinogruppe,
* wobei es sich bei i um eine ganze Zahl von 1 bis n handelt,
- es sich bei Y um eine NR_{b}R_{c}-Gruppe handelt, wobei R_{b} und R_{c} die Bedeutung haben, die vorstehend für Rⁱ angegeben wurde,
wobei die Helix die folgenden kennzeichnenden Eigenschaften aufweist:
- sie weist eine gleichmäßige Rechtswindung auf, die zwischen 4,9 Å und 5,3 Å liegt, wobei sie insbesondere gleich 5 Å ist,
- sie umfasst pro Windung 2,4 bis 2,6 Reste, wobei die Anzahl der Reste pro Windung insbesondere 2,5 beträgt,
- ihr Innendurchmesser, der ausgehend von den Atommitten berechnet wurde, liegt zwischen 3,8 Å und 4,6 Å und ist insbesondere gleich 4,2 Å,
- ihr Innendurchmesser, der ausgehend von der Vander-Waals-Oberfläche berechnet wurde, liegt zwischen 1,4 Å und 1,8 Å und ist insbesondere gleich 1,6 Å,

8. Verbindung nach Anspruch 7, welche der folgenden Formel entsprechen:

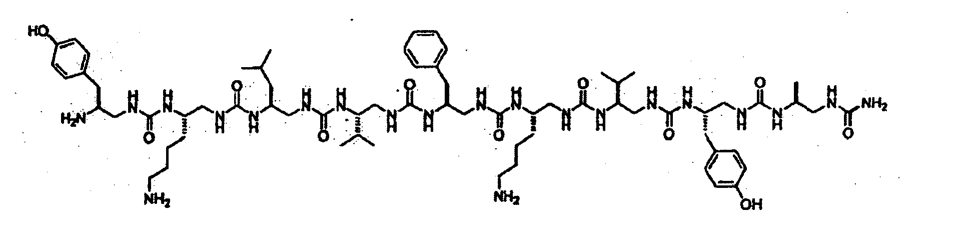

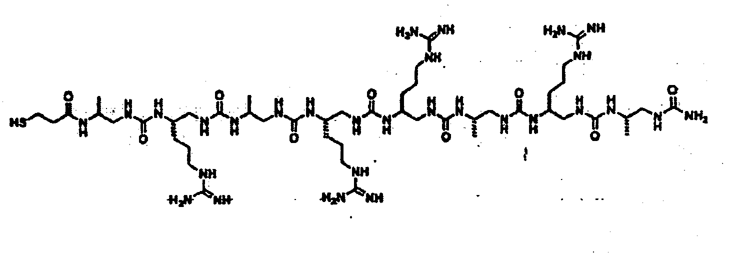

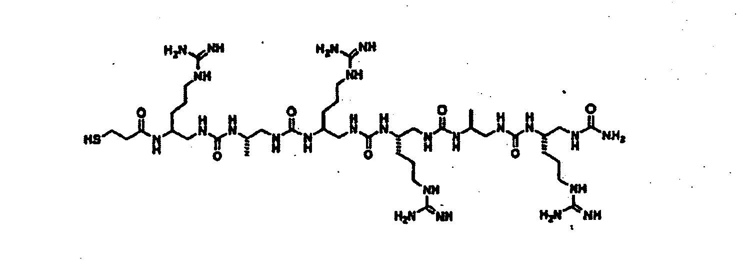

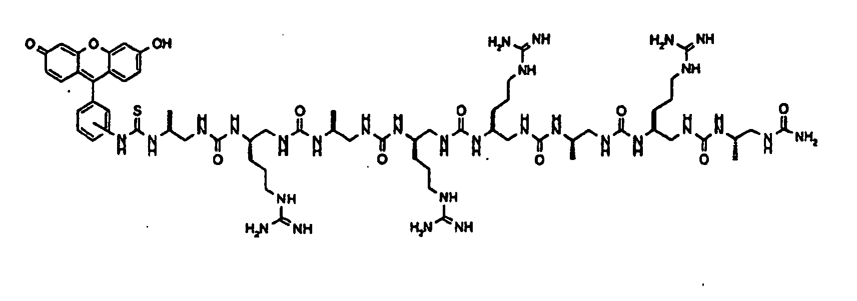

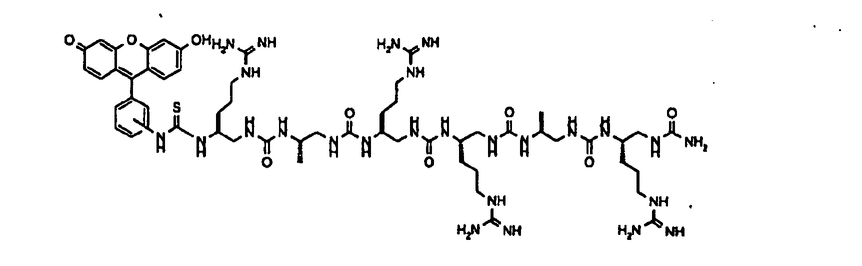

9. Zusammensetzung, die mindestens eine Verbindung nach Anspruch 7 sowie mindestens ein Lösemittel enthält, wobei das Lösemittel die Eigenschaft hat, den Molekülen der Verbindung eine Helixform zu verleihen, was auch teilweise geschehen kann, falls ein Konformationsgleichgewicht zwischen den Molekülen der Verbindung, die in Helixform vorliegen, und den Molekülen der Verbindung, die in nicht helixartiger Form vorliegen, besteht, und wobei es insbesondere aus den Alkoholen, aus Pyridin, aus Acetonitril, aus Dimethylsulfoxid, aus Wasser in Gegenwart oder Abwesenheit von Mizellen oder Lipiden gewählt ist, wobei es vorteilhafterweise aus Wasser in Gegenwart oder Abwesenheit von Mizellen oder Lipiden, aus Methanol, aus Ethanol, aus Isopropanol, aus Trifluorethanol, aus Hexafluorisopropanol, aus Dimethylsulfoxid oder einer beliebigen Mischung dieser Lösemittel gewählt ist.

10. Pharmazeutische Zusammensetzung, **dadurch gekennzeichnet, dass** sie als Wirkstoff mindestens eine der Verbindungen nach einem der Ansprüche 2 bis 6 umfasst, in Verbindung mit einem pharmazeutisch unbedenklichen Vektor.

11. Pharmazeutische Zusammensetzung nach Anspruch 10, **dadurch gekennzeichnet, dass** sie pro Einzeldosis 10 bis 2000 mg mindestens einer der Verbindungen gemäß einem der Ansprüche 2 bis 6 enthält.

12. Pharmazeutische Zusammensetzung, **dadurch gekennzeichnet, dass** sie die Verbindung nach dem Anspruch 8 umfasst, in Verbindung mit einem pharmazeutisch unbedenklichen Vektor.

13. Pharmazeutische Zusammensetzung, **dadurch gekennzeichnet, dass** sie als pharmazeutischen Vektor mindestens eine der Verbindungen nach einem der Ansprüche 2 bis 8 umfasst, in Verbindung mit einer pharmazeutisch wirksamen Substanz.

14. Verwendung einer Verbindung nach einem der Ansprüche 2 bis 8 als pharmazeutischer Vektor.

15. Festphasenherstellungsverfahren für die Verbindungen nach Formel (I) nach Anspruch 2, ausgehend von voraktivierten Monomeren nach der Formel GP-A-W, wobei:
- GP eine Schutzgruppe ist, die aus Fmoc, Teoc, Phthalimid, Alloc, BOC und Cbz gewählt ist,
- A der Begriffsbestimmung in Anspruch 2 entspricht,
- W aus der Gruppe W-H stammt, die aus Folgendem gewählt ist: N-Hydroxysuccinimid, Phenol, Pentafluorphenol, Pentachlorphenol, p-Nitrophenol, 2,4-Dinitrophenol, 2,4,5-Trichlorphenol, 2,4-Dichlor-6-nitrophenol, Hydroxy-1,2,3-benzotriazol, 1-Oxo-2-hydroxydihydrobenzotriazin (HODhbt), 7-Aza-1-hydroxybenzotriazol (HOAt), 4-Aza-1-hydroxybenzotriazol (4-HOAt), Imidazol, Tetrazol sowie dem Harz WANG, wobei es sich vorzugsweise um N-Hydroxysuccinimid oder p-Nitrophenol handelt,
**dadurch gekennzeichnet, dass** es die folgenden Schritte umfasst:
a) einen Schritt, bei dem GP-A-W gemäß der obigen Begriffsbestimmung an die funktionelle Aminogruppe eines Trägerstoffes gekoppelt wird, wobei dieser Schritt es ermöglicht, die -A-GP-Gruppe auf die funktionelle Aminogruppe des Trägerstoffes zu pfropfen,
b) einen Schritt, bei dem die GP-Gruppe unter geeigneten Bedingungen abgespalten wird,
c) die Wiederholung der Schrittfolge a) bis b), solange bis n A-Gruppen auf den Trägerstoff gepfropft worden sind,
d) einen Schritt, bei dem die Gruppen RₐCO, RₐOCO, RₐNHCO oder RₐSO₂ eingeführt werden, wobei Rₐ der Begriffsbestimmung in Anspruch 2 entspricht,
e) einen Schritt, bei dem die Verbindung nach Formel (I) mit Hilfe geeigneter Mittel vom Träger abgespalten wird, wodurch das Harz losgelöst und die Gruppe Y, die der Begriffsbestimmung in Anspruch 2 entspricht, freigesetzt werden kann.

16. Herstellungsverfahren nach Anspruch 15, **dadurch gekennzeichnet, dass** der Schritt a) in Gegenwart oder in Abwesenheit einer tertiären Base wie etwa DIEA, Collidin, NMM oder Lutidin sowie in Gegenwart oder in Abwesenheit eines Katalysators wie etwa HOBt durchgeführt werden kann.

17. Herstellungsverfahren nach Anspruch 15, **dadurch gekennzeichnet, dass** der Schritt a) in einem Lösemittel wie etwa DMF, CH₂Cl₂, THF oder N-Methylpyrrolidon durchgeführt werden kann.

18. Herstellungsverfahren nach Anspruch 15, **dadurch gekennzeichnet, dass** die es sich bei der GP-Gruppe um eine Fmoc-Gruppe handelt.

19. Flüssigphasenherstellungsverfahren für die Verbindungen nach Formel (I) nach Anspruch 2, ausgehend von voraktivierten Monomeren nach der Formel GP-A-W, wobei:
- GP eine Schutzgruppe ist, die aus Fmoc, Alloc, BOC und Cbz gewählt ist,
- A der Begriffsbestimmung in Anspruch 2 entspricht,
- W aus der Gruppe W-H stammt, die aus Folgendem gewählt ist: N-Hydroxysuccinimid, Phenol, Pentafluorphenol, Pentachlorphenol, p-Nitrophenol, 2,4-Dinitrophenol, 2,4,5-Trichlorphenol, 2,4-Dichlor-6-nitrophenol, Hydroxy-1,2,3-benzotriazol, 1-Oxo-2-hydroxydihydrobenzotriazin (HODhbt), 7-Aza-1-hydroxybenzotriazol (HOAt), 4-Aza-1-hydroxybenzotriazol (4-HOAt), Imidazol und Tetrazol, wobei es sich vorzugsweise um N-Hydroxysuccinimid oder p-Nitrophenol handelt,
**dadurch gekennzeichnet, dass** es die folgenden Schritte umfasst:
a) einen Schritt, bei dem GP-A-W gemäß der obigen Begriffsbestimmung an die funktionelle Aminogruppe der Gruppe A des Derivats H-A-Y gekoppelt wird,
b) einen Schritt, bei dem die GP-Gruppe unter geeigneten Bedingungen abgespalten wird,
c) die Wiederholung der Schrittfolge a) bis b), solange bis n A-Gruppen auf die oben genannte funktionelle Aminogruppe gepfropft worden sind,
d) einen Schritt, bei dem die Gruppen RₐCO, RₐOCO, RₐNHCO oder RₐSO₂ eingeführt werden, wobei Rₐ der Begriffsbestimmung in Anspruch 2 entspricht,
e) einen Schritt, bei dem die Verbindung nach Formel (I) mit Hilfe geeigneter Mittel abgespalten wird.

20. Herstellungsverfahren für die Verbindungen nach Anspruch 7, bei welchem zu diesem Zwecke eine der Verbindungen gemäß der Begriffsbestimmung in Anspruch 2 oder eine der Verbindungen nach den Formeln (VIIc) oder (VIId) mit einem Lösemittel wie etwa Methanol, Ethanol, Trifluorethanol, Hexafluorisopropanol, Wasser oder mit biologischen Puffern, bei denen es sich etwa um Phosphatpuffer handeln kann oder auch nicht, oder mit einem mizellären oder lipidischen Medium in Kontakt gebracht werden.
